# EUROPÄISCHE PATENTSCHRIFT

(11) **EP 2 046 916 B1**
(45) Veröffentlichungstag und Bekanntmachung des Hinweises auf die Patenterteilung: **07.10.2015**
(21) Anmeldenummer: 07786395.9
(22) Anmeldetag: 27.07.2007
(51) Int. Cl.: C07F 15/00, C09K 11/06

(54) **OXAZOL-TRIPLETT-EMITTER FÜR OLED-ANWENDUNGEN**
OXAZOLE TRIPLET EMITTERS FOR OLED APPLICATIONS
ÉMETTEUR TRIPLET D'OXAZOLE POUR APPLICATIONS OLED

(30) Priorität: 28.07.2006 DE 102006035018
(43) Veröffentlichungstag der Anmeldung: 15.04.2009
(73) Patentinhaber: Merck Patent GmbH, 64293 Darmstadt (DE)
(72) Erfinder: YERSIN, Harmut, 93161 Sinzing (DE); REISER, Oliver, 93059 Regensburg (DE); LI, Luo Qun, 93049 Regensburg (DE); EIBAUER, Stefan, 09469 Weinheim (DE)
(86) Internationale Anmeldenummer: PCT/EP2007/006683
(87) Internationale Veröffentlichungsnummer: WO 2008/012103

(56) Entgegenhaltungen:
- DE-A1- 4 112 793
- GB-A- 1 436 230
- US-A- 5 792 568
- DENMARK S E ET AL: "CYCLOPROPANATION WITH DIAZOMETHANE AND BIS(OXAZOLINE) PALLADIUM (II) COMPLEXES" JOURNAL OF ORGANIC CHEMISTRY, AMERICAN CHEMICAL SOCIETY. EASTON, US, Bd. 62, Nr. 10, 16. Mai 1997 (1997-05-16), Seiten 3375-3389, XP000689793 ISSN: 0022-3263
- TARRAGA A ET AL: "Synthesis and electrochemical study of novel oxazolo-ferrocene derivatives displaying redox-switchable character" TETRAHEDRON, ELSEVIER SCIENCE PUBLISHERS, AMSTERDAM, NL, Bd. 57, Nr. 31, 30. Juli 2001 (2001-07-30), Seiten 6765-6774, XP004275012 ISSN: 0040-4020

## Beschreibung

Die vorliegende Erfindung betrifft Verbindungen, die insbesondere als Liganden Verwendung finden können, Komplexe sowie Licht emittierende Vorrichtungen und insbesondere organische Licht emittierende Vorrichtungen (OLED). Insbesondere betrifft die Erfindung den Einsatz lumineszierender Oxazol-Chelat-Metallkomplexe als Emitter in solchen Vorrichtungen.

OLEDs (Organic Light Emitting Devices oder Organic Light Emitting Diodes) stellen eine neue Technologie dar, welche die Bildschirm- und Beleuchtungstechnik dramatisch verändern wird. OLEDs bestehen vorwiegend aus organischen Schichten, die auch flexibel und kostengünstig zu fertigen sind. OLED-Bauelemente lassen sich großflächig als Beleuchtungskörper, aber auch klein als Pixels für Displays gestalten.

Einen Überblick über die Funktion von OLEDs findet sich beispielsweise bei H. Yersin, Top. Curr. Chem. 2004, 241,1 und in H. Yersin, "Highly Efficient OLEDs with Phosphorescent Materials", Wiley-VCH 2006.

Die Funktion von OLEDs ist auch beschrieben worden in C. Adachi et al., Appl. Phys. Lett. 2001, 78, 1622; X.H. Yang et al., Appl. Phys. Lett. 2004, 84, 2476; J. Shinar, "Organic Light-Emitting Devices - A Survey", AlP-Press, Springer, New York 2004; W. Sotoyama et al., Appl. Phys. Lett. 2005, 86, 153505; S. Okada et al., Dalton Trans., 2005, 1583 sowie Y. -L. Tung et al., J. Mater. Chem., 2005, 15, 460-464.

Seit den ersten Berichten über OLEDs (s. z.B. Tang et al., Appl. Phys. Lett. 51 (1987) 913) sind diese Vorrichtungen insbesondere im Hinblick auf die eingesetzten Emittermaterialien weiter entwickelt worden, wobei in jüngster Zeit insbesondere sogenannte Triplett- oder phosphoreszierende Emitter von Interesse sind.

Gegenüber herkömmlichen Technologien, wie etwa Flüssigkristall-Displays (LCDs), Plasma-Displays oder Kathodenstrahlröhren (CRTs) weisen OLEDs zahlreiche Vorteile auf, wie zum Beispiel eine geringe Betriebsspannung, eine flache Bauweise, hoch-effizient selbst-leuchtende Pixel, einen hohen Kontrast und eine gute Auflösung sowie die Möglichkeit, alle Farben darzustellen. Weiterhin emittiert ein OLED Licht beim Anlegen elektrischer Spannung anstelle es nur zu modulieren. Während dem OLED bereits zahlreiche Anwendungen erschlossen sind und auch neue Anwendungsgebiete eröffnet wurden, besteht immer noch Bedarf an verbesserten OLEDs und insbesondere an verbesserten Triplett-Emittermaterialien. Bei den bisherigen Lösungen treten insbesondere Probleme bei der Langzeitstabilität, der thermischen Stabilität sowie der chemischen Stabilität gegenüber Wasser und Sauerstoff auf. Weiterhin zeigen viele Emitter nur eine geringe Sublimationsfähigkeit. Weiterhin sind mit bisher bekannten Emittermaterialien oftmals wichtige Emissionsfarben nicht verfügbar. Oftmals sind auch hohe Effizienzen bei hohen Stromdichten oder hohe Leuchtdichten nicht erreichbar. Schließlich bestehen bei vielen Emittermaterialien Probleme hinsichtlich der fertigungstechnischen Reproduzierbarkeit.

Weiterhin wurde festgestellt, dass die Lichtausbeute für OLEDs mit Metallorganischen Substanzen, sogenannten Triplett-Emittern wesentlich größer sein kann als für rein organische Materialien. Aufgrund dieser Eigenschaft kommt der Weiterentwicklung von metallorganischen Materialien ein wesentlicher Stellenwert zu. Triplett-Emitter sind beispielsweise beschrieben in WO 2004/017043 A2 (Thompson), WO 2004/016711 A1 (Thompson), WO 03/095587 (Tsuboyama), US 2003/0205707 (Chi-Ming Che), US 2002/0179885 (Chi-Ming Che), US 2003/186080 A1 (J. Kamatani), DE 103 50 606 A1 (Stößel), DE 103 38 550 (Bold), DE 103 58 665 A1 (Lennartz).

US 5 792 568 A zeigt die Verwendung von Bis(oxazol)-Verbindungen in organischen elektrolumineszenten Vorrichtungen.

Eine Aufgabe der vorliegenden Erfindung war es, neue Emittermaterialien, insbesondere für OLEDs sowie neue Licht emittierende Vorrichtungen bereitzustellen, welche die Nachteile des Standes der Technik zumindest teilweise überwinden und welche insbesondere sehr stabil sind und gut sublimiert werden können.

Diese Aufgabe wird erfindungsgemäß gelöst durch Komplexe der Formel (I) oder (II) 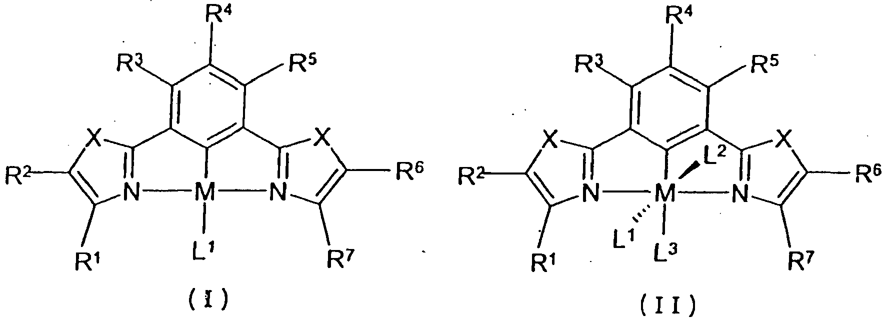 worin
M ausgewählt ist aus einem Element der Gruppe 6 bis 11 der 2. oder 3. Periode der Übergangsmetalle, insbesondere aus Mo, Ru, Rh, Pd, Ag, W, Re, Os, Ir, Pt und Au,
X ein Element der Gruppe 15 oder 16 des Periodensystems und insbesondere Sauerstoff darstellt,
R¹ bis R⁷ jeweils unabhängig voneinander Wasserstoff, Halogen, R', O-R' oder N-R'R" darstellen, worin
R' eine Kohlenwasserstoffgruppe darstellt, welche gegebenenfalls Heteroatome enthalten kann und R" für H steht oder eine für R' angegebene Bedeutung besitzt, wobei zwei oder mehrere Gruppen R¹ bis R⁷ auch annelierte Ringsysteme bilden können und
L¹, L² und L³ jeweils unabhängig voneinander einen negativ geladenen oder neutralen Liganden darstellen, wobei zwei oder mehr der Liganden L¹, L² und L³ miteinander verbunden sein können.

Bei den erfindungsgemäßen Komplexen handelt es sich insbesondere um lumineszierende Verbindungen. Die Komplexe weisen ein Zentralatom auf, welches ausgewählt wird aus einem Element der Gruppe 6 - 11 der 2. und 3. Periode der Übergangsmetalle, insbesondere aus Mo, Tc, Ru, Rh, Pd, Ag, W, Re, Os, Ir, Pt oder Au, bevorzugt aus Mo, Ru, Rh, Pd, Ag, W, Re, Os, Ir, Pt oder Au, noch mehr bevorzugt aus Pt, Pd, Ru, Os oder Ir, vorzugsweise aus Pt oder Pd. Das Zentralatom liegt bevorzugt in den Oxidationsstufen 0 bis +4 vor. Besonders bevorzugt ist das Zentralatom Pt(II), Ir(III) oder Pd(II). Erfindungsgemäß ist das Zentralatom vierfach oder sechsfach koordiniert. Bei den erfindungsgemäßen Komplexen handelt es sich bevorzugt um Komplexe mit einem einzigen Metall-Zentralatom.

Die erfindungsgemäßen Komplexe enthalten weiterhin einen Liganden der Formel (III) 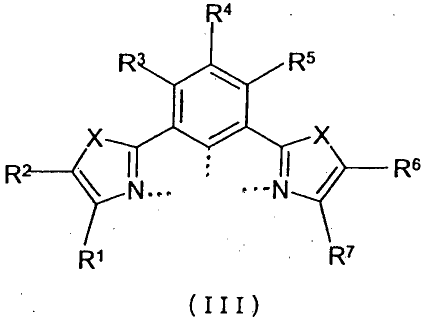 der hierin auch als Oxazolligand bezeichnet wird. Bei dem Oxazolliganden handelt es sich um einen dreizähnigen Liganden. Die Gruppierung X stellt dabei ein Element der Gruppe 15 (also N, P, AS, Sb oder Bi), insbesondere Stickstoff, oder der Gruppe 16 (also O, S, Se, Te oder Po), insbesondere Sauerstoff oder Schwefel, des Periodensystems der Elemente dar. Am meisten bevorzugt stellt X Sauerstoff dar. Für den Fall, dass X ein Element der Gruppe 15 darstellt, ist die freie Valenz geeignet gebunden, z.B. mit H, Alkyl, Aryl etc.

Der Oxazolligand enthält weiterhin Reste R¹ bis R⁷, welche jeweils unabhängig voneinander Wasserstoff, Halogen oder eine Kohlenwasserstoffgruppe darstellen, welche ggf. Heteroatome enthalten und/oder substituiert sein kann, sowie über Sauerstoff oder Stickstoff an das Grundgerüst gebundene Kohlenwasserstoff gruppen, welche ebenfalls ggf. Heteroatome enthalten und/oder substituiert sein können.

Die Heteroatome werden insbesondere ausgewählt aus O, S, N, P, Si, Se, F, Cl, Br und/oder I. Die Reste R¹ bis R⁷ bzw. R' oder R" weisen vorzugsweise jeweils 0 bis 50, insbesondere 0 bis 10, und noch mehr bevorzugt 0 bis 5 Heteroatome auf. In manchen Ausführungsformen weisen die Reste R¹ bis R⁷ bzw. R' oder R" jeweils mindestens 1, insbesondere mindestens 2 Heteroatome auf. Die Heteroatome können dabei im Gerüst oder als Teil von Substituenten vorliegen. In einer Ausführungsform handelt es sich bei den Resten R¹ bis R⁷ oder R' oder R" um eine Kohlenwasserstoffgruppe, welche eine oder mehrere funktionelle Gruppen aufweist. Geeignete funktionelle Gruppen sind beispielsweise Halogen, insbesondere F, Cl, Br oder I, Alkyl, insbesondere C₁ bis C₂₀, noch mehr bevorzugt C₁ bis C₆ Alkyl, Aryl, O-Alkyl, O-Aryl, S-Aryl, S-Alkyl, P-Alkyl₂, P-Aryl₂, N-Alkyl₂ oder N-Aryl₂ oder andere donor- oder Akzeptor-Gruppen. In vielen Fällen ist es bevorzugt, dass wenigstens einer der Reste R¹ bis R⁷ oder R' oder R" zur Erhöhung der Flüchtigkeit des Komplexes wenigstens ein Fluor enthält.

Bevorzugt handelt es sich hierbei bei einer Kohlenwasserstoffgruppe um eine Alkyl-, Alkenyl-, Alkinyl-, Aryl- oder Heteroaryl-Gruppe. In einer weiteren bevorzugten Ausführungsform ist wenigstens einer der Reste R¹ bis R⁷ O-R' oder N-R'R", wobei R' wiederum eine Kohlenwasserstoffgruppe darstellt, die ein oder mehrere Heteroatome enthalten kann. Bevorzugt ist R' Alkyl, Aryl, Heteroaryl, welches ggf. eine oder mehrere funktionelle Gruppen, wie oben angegeben, enthalten kann. R" ist H oder hat eine der für R' angegebenen Bedeutungen. Am meisten bevorzugt stellen R¹ bis R⁷ ein oder mehrere Reste von H, Phenyl, t-Butyl, COO-Ethyl oder O-Ethyl dar.

In den erfindungsgemäßen Komplexen ist es aber auch möglich, dass zwei oder mehrere Reste aus R¹ bis R⁷ zusammen ein anneliertes Ringsystem bilden.

Falls nicht anders angegeben, bezeichnet der Ausdruck Alkyl- oder Alk-, wie hierin verwendet, jeweils unabhängig bevorzugt eine C₁ - C₂₀, insbesondere eine C₁ - C₆ Kohlenwasserstoffgruppe. Der Ausdruck Aryl- bezeichnet bevorzugt ein aromatisches System mit 5 bis z.B. 20 C-Atomen, insbesondere mit 6 bis 10 C-Atomen, wobei gegebenenfalls C-Atome durch Heteroatome ersetzt sein können (z.B. N, S, O).

Der Komplex enthält weiterhin Liganden L¹ bzw. Liganden L¹, L² und L³. L¹, L² und L³ stehen jeweils unabhängig für negativ geladene oder neutrale Liganden, bevorzugt für Halogene, insbesondere Chlorid oder Bromid,für Pseudohalogene, insbesondere Thiocyanat, Cyanat oder Cyanid. Weiterhin bevorzugt sind Liganden L¹, L² oder L³, die über ein Element der 16. Gruppe des Periodensystems, insbesondere über Sauerstoff oder Schwefel an das Zentralatom gebunden sind (z.B. Alkoxide oder Thiolate), bevorzugt O-R' oder S-R', wobei R' die hierzu angegebenen Bedeutungen haben kann.

In einer weiteren Ausführungsform bevorzugt sind Liganden, die über ein Element der 15. Gruppe des Periodensystems, insbesondere über Stickstoff, Phosphor oder Arsen (z.B. Nitrile, Amine, Phosphane, Arsane) gebunden sind, insbesondere NR'R", PR'R" oder AsR'R", oder über ein Element der 14. Gruppe, insbesondere Kohlenstoff (z.B. Cyanid, Isonitrile, Acetylide (C≡C)ₙR⁸, wobei n = 1 - 10 und R⁸ = Alkyl oder Aryl, ggf. substituiert, Tri (alkyl)silyl). Im Fall von Koordinationszahlen größer als 4 können die Liganden L¹⁻³ voneinander unabhängig oder miteinander verbunden, d.h. mehrzähnig sein. Die mehrzähnigen Liganden können wieder neutral oder negativ geladen sein. Bevorzugt sind bespielsweise Bis- bzw. Tris(pyrazolyl) borate als negativ geladene Liganden oder Bis- und Tris(phosphane), Diamine etc. als Beispiele für neutrale Liganden. Bevorzugt bilden L¹, L² und L³ zusammen einen Liganden

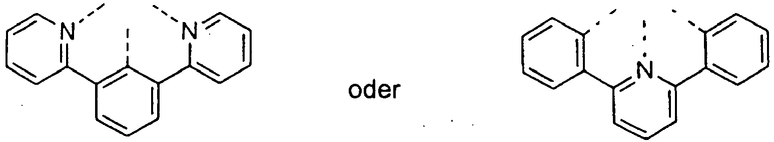

In einer bevorzugten Ausführungsform bilden L¹, L² und L³ zusammen wiederum einen Liganden der Formel (III).

Besonders bevorzugt sind die Komplexe
- 5,5'-Di-*tert*-butyl-2,2'-*m*-phenylen-bis-oxazol-2-platinum(II)bromid,
- 5,5'-Diphenyl-2,2'-m-phenylen-bis-oxazol-2-platinum(II)bromid,
- 2,6-bis(5-ethoxy-oxazol-2-yl)phenyl-bromoplatinum(II),
- 2-(4-methodycarbonyl-oxazol-2-yl)-6-(5-ethoxy-oxazol-2-yl)phenyl-bromoplatinum(II),
- (3-Oxo-3-ethoxypropynyl)-[2,6-bis(5-ethoxy-oxazol-2-yl)phenyl]-platinum (II),
- (3-hydroxylpropynyl)-[2,6-bis(5-ethoxy-oxazol-2-yl)phenyl]-platinum(II),
- 5,5'-Di-*tert*-butyl-2,2'-*m*-phenylene-bis-oxazol-2-palladium(II)bromid,
- 5,5'-Diphenyl-2,2'-*m*-phenylen-bis-oxazol-2-palladium(II)bromid sowie

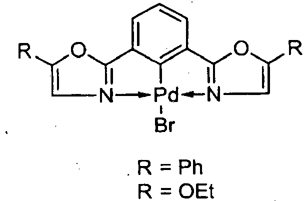

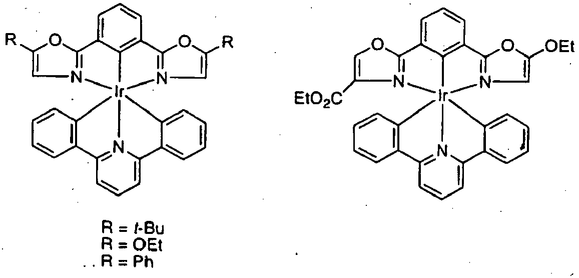

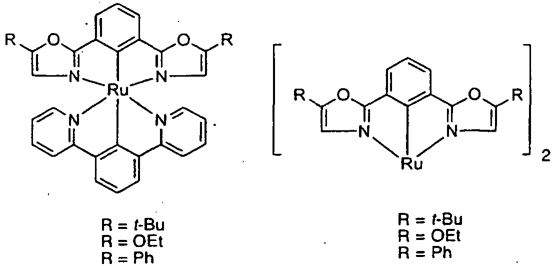

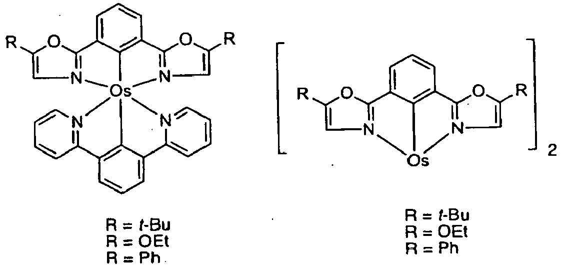

Die erfindungsgemäßen Verbindungen weisen völlig neue Molekülstrukturen mit dreizähniger Ligand-Metall-Bindung auf. Die erfindungsgemäßen Komplexe mit dreibindigen Chelatliganden sind insbesondere thermisch sehr stabil. Sie sind daher aus technischer Sicht hervorragend zur Sublimation geeignet.

Die Erfindung betrifft weiterhin Verbindungen der Formel (IV). 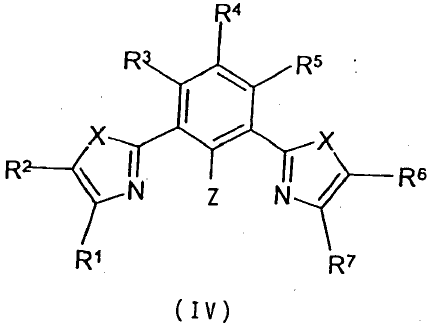 worin
X und R¹ bis R⁷ wie hierin definiert sind, und
Z eine Abgangsgruppe darstellt, insbesondere H oder Halogen, z.B. F, Cl, Br oder J, bevorzugt Br.

Diese Verbindungen eignen sich insbesondere als Liganden. Komplexe umfassend aus Verbindungen der Formel (IV) gebildete Liganden der Formel III sind aufgrund deren Drei-Bindigkeit thermisch stabil.

Besonders bevorzugt sind folgende Verbindungen der Formel (IV)

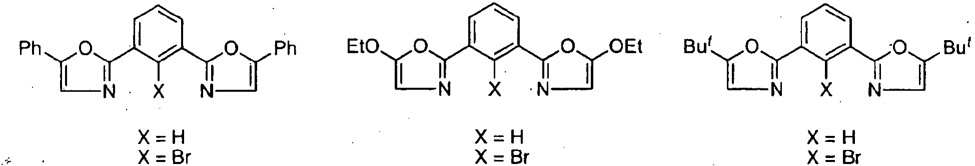

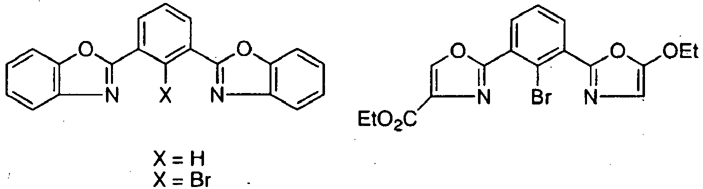

Überraschenderweise wurde festgestellt, dass durch den erfindungsgemäßen Einsatz der Komplexe der Formel (I) oder (II) in der Emitterschicht Licht emittierende Vorrichtungen erhalten werden können, die hervorragende Eigenschaften aufweisen. Die erfindungsgemäßen eingesetzten Verbindungen der Formel (I) oder (II) sind insbesondere stabil bezüglich einer Sublimation und eignen sich daher für die Herstellung von OLEDs mittels Vakuumabscheidung. Weiterhin zeigen sie bei Anwendung als Emitter in OLEDs eine Elektrophosphoreszenz mit hoher Effizienz und Helligkeit. Insbesondere zeigen die erfindungsgemäß eingesetzten Verbindungen hohe Quantenausbeuten. Die Komplexe können zudem durch Substitution oder/und Veränderung der Liganden variiert werden, wodurch sich vielfältige Möglichkeiten zur Modifizierung bzw. Steuerung der Emissionseigenschaften (z.B. Farbe, Quantenausbeute, Abklingzeit usw.) ergeben.

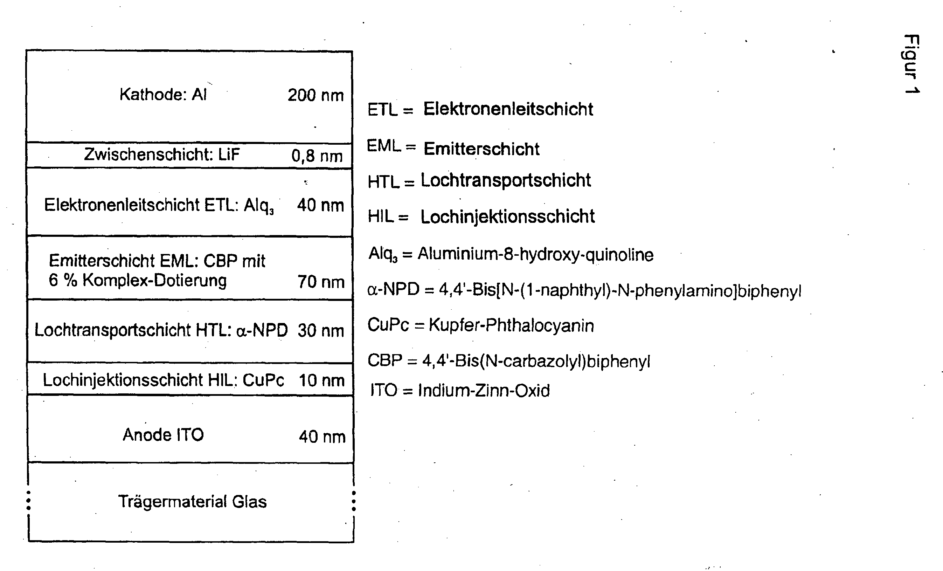

Die Funktionsweise einer Ausführungsform der erfindungsgemäßen Licht emittierenden Vorrichtungen ist schematisch in Figur 1 gezeigt. Die Vorrichtung umfasst mindestens eine Anode, eine Kathode und eine Emitterschicht. Vorteilhafterweise wird eine oder beide der als Kathode oder Anode verwendeten Elektroden transparent ausgestaltet, sodass das Licht durch diese Elektrode emittiert werden kann. Bevorzugt wird als transparentes Elektrodenmaterial Indium-Zinn-Oxid (ITO) verwendet. Besonders bevorzugt wird eine transparente Anode eingesetzt. Die andere Elektrode kann ebenfalls aus einem transparenten Material ausgebildet sein, kann aber auch aus einem anderen Material mit geeigneter Elektronenaustrittsarbeit gebildet sein, falls Licht nur durch eine der beiden Elektroden emittiert werden soll. Vorzugsweise besteht die zweite Elektrode, insbesondere die Kathode, aus einem Metall mit niedriger Elektronenaustrittsarbeit und guter elektrischer Leitfähigkeit, beispielsweise aus Aluminium, oder Silber, oder einer Mg/Ag-oder einer Ca/Ag-Legierung. Zwischen den beiden Elektroden ist eine Emitterschicht angeordnet. Diese kann in direktem Kontakt mit der Anode und der Kathode sein, oder in indirektem Kontakt, wobei indirekter Kontakt bedeutet, dass zwischen der Kathode oder Anode und der Emitterschicht weitere Schichten enthalten sind, sodass die Emitterschicht und die Anode oder/und Kathode sich nicht berühren, sondern über weitere Zwischenschichten elektrisch miteinander in Kontakt stehen. Bei Anlegen einer Spannung, beispielsweise einer Spannung von 2 - 20 V, insbesondere von 5 - 10 V, treten aus der Kathode, beispielsweise einer leitenden Metallschicht, z.B. aus einer Aluminium-Kathode negativ geladene Elektronen aus und wandern in Richtung der positiven Anode. Von dieser Anode ihrerseits wandern positive Ladungsträger, sogenannte Löcher, in Richtung der Kathode. In der zwischen der Kathode und Anode angeordneten Emitterschicht befinden sich erfindungsgemäß die metallorganischen Komplexe der Formeln (I) und (II) als Emitter-Moleküle. An den Emitter-Molekülen oder in deren Nähe rekombinieren die wandernden Ladungsträger, also ein negativ geladenes Elektron und ein positiv geladenes Loch, und führen dabei zu neutralen, aber energetisch angeregten Zuständen der Emitter-Moleküle. Die angeregten Zustände der Emitter-Moleküle geben dann ihre Energie als Lichtemission ab.

Die erfindungsgemäßen Licht emittierenden Vorrichtungen können, soweit die Emittermaterialien sublimierbar sind, über Vakuumdeposition hergestellt werden. Alternativ ist auch ein Aufbau über nass-chemische Auftragung möglich, beispielsweise über Spin-Coating-Verfahren, über Inkjet-Printen oder über Siebdruckverfahren. Der Aufbau von OLED-Vorrichtungen wird beispielsweise in US2005/0260449 A1 sowie in WO 2005/098988 A1 ausführlich beschrieben.

Die erfindungsgemäßen Licht emittierenden Vorrichtungen können mittels der Vakuum-Sublimations-Technik gefertigt werden und mehrere weitere Schichten enthalten, insbesondere eine Elektroneninjektionsschicht und eine Elektronen-Leitungsschicht (z.B. Alq₃ = AI-8-hydroxychinolin oder B-Alq = Al-bis(2-methyl-8-hydroxychinolato)-4-phenylphenolat) und/oder eine Loch-Injektions- (z.B. CuPc) und Loch-Leitungsschicht oder Loch-Leitungsschicht (z.B. α-NPD). Es ist aber auch möglich, dass die Emitterschicht Funktionen der Loch- bzw. Elektronen-Leitungsschicht übernimmt (geeignete Materialien sind auf den Seiten 7/8 erläutert).

Die Emitterschicht besteht vorzugsweise aus einem organischen Matrixmaterial mit für die jeweilige Emissionsfarbe (T₁-Lage) ausreichend großem Singulett S₀ - Triplett T₁-Energieabstand, z.B. aus UGH, PVK (Polyvinylcarbazol), CBP (4,4'-Bis(9-carbazolyl)biphenyl) oder anderen Matrixmaterialien. In dieses Matrixmaterial wird der Emitter-Komplex eindotiert, z.B. bevorzugt mit 1 bis 10 Gewichtsprozent.

Die Emitterschicht kann auch ohne Matrix realisiert werden, indem der entsprechende Komplex als 100%-Material aufgebracht wird. Eine entsprechende Ausführungsform ist weiter unten beschrieben.

In einer besonders bevorzugten Ausführungsform weist die erfindungsgemäße Licht emittierende Vorrichtung zwischen der Kathode und der Emitterschicht oder einer Elektronenleiterschicht noch eine CsF Zwischenschicht auf. Diese Schicht weist insbesondere eine Dicke von 0,5 nm bis 2 nm, bevorzugt von ca. 1 nm auf. Diese Zwischenschicht bewirkt vorwiegend eine Reduzierung der Elektronenaustrittsarbeit.

Weiterhin bevorzugt wird die Licht emittierende Vorrichtung auf einem Substrat aufgebracht, beispielsweise auf einem Glassubstrat.

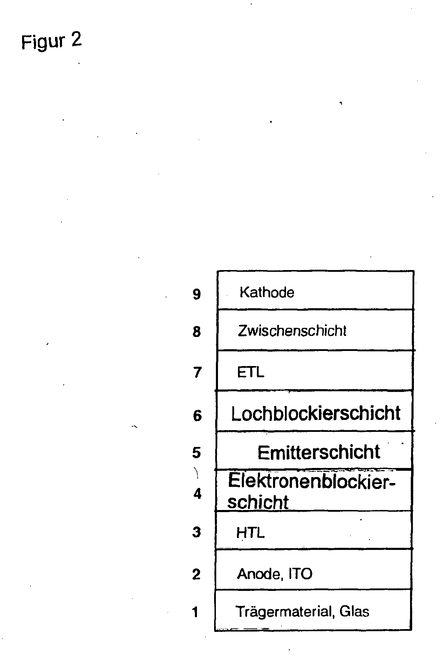

In einer besonders bevorzugten Ausführungsform umfasst ein OLED-Aufbau für einen sublimierbaren erfindungsgemäßen Emitter neben einer Anode, Emitterschicht und Kathode auch noch wenigstens eine, insbesondere mehrere und besonders bevorzugt alle der nachfolgend genannten und in Figur 2 dargestellten Schichten.

Der gesamte Aufbau befindet sich vorzugsweise auf einem Trägermaterial, wobei hierfür insbesondere Glas oder jedes andere feste oder flexible durchsichtige Material eingesetzt werden kann. Auf dem Trägermaterial wird die Anode angeordnet, beispielsweise eine Indium-Zinn-Oxid-Anode (ITO). Auf die Anode und zwischen Emitterschicht und Anode wird eine Lochtransportschicht (HTL, Hole Transport Layer) angeordnet, beispielsweise α-NPD (N,N'-Diphenyl-N,N'-bis(1-methyl)-1,1'-biphenyl-4,4'-diamin). Die Dicke der Lochtransportschicht beträgt vorzugsweise 10 bis 100 nm, insbesondere 30 bis 50 nm. Zwischen der Anode und der Lochtransportschicht können weitere Schichten angeordnet sein, die die Lochinjektion verbessern, z.B. eine Kupfer-Phthalocyanin (CuPc)-Schicht. Diese Schicht ist bevorzugt 5 bis 50, insbesondere 8 bis 15 nm dick. Auf die Lochtransportschicht und zwischen Lochtransport- und Emitterschicht wird vorzugsweise eine Elektronenblockierschicht aufgetragen, die dafür sorgt, dass der Elektronentransport zur Anode unterbunden wird, da ein solcher Strom nur Ohm'sche Verluste verursachen würde. Die Dicke dieser Elektronenblockierschicht beträgt vorzugsweise 10 bis 100 nm, insbesondere 20 bis 40 nm. Auf diese zusätzliche Schicht kann insbesondere dann verzichtet werden, wenn die HTL-Schicht bereits intrinsisch ein schlechter Elektronenleiter ist.

Bei der nächsten Schicht handelt es sich um die Emitterschicht, die das erfindungsgemäße Emittermaterial enthält oder aus diesem besteht. In der Ausführungsform unter Verwendung von sublimierbaren Emittern werden die Emittermaterialien bevorzugt durch Sublimation aufgetragen. Die Schichtdicke beträgt vorzugsweise zwischen 40 nm und 200 nm, insbesondere zwischen 70 nm und 100 nm. Das erfindungsgemäße Emittermaterial kann auch gemeinsam mit anderen Materialien, insbesondere mit Matrixmaterialien co-verdampft werden. Für im Grünen oder Roten emittierende erfindungsgemäße Emittermaterialien eignen sich gängige Matrixmaterialien wie CBP (4,4'-Bis(N-carbazolyl)biphenyl). Es ist aber für Komplexe nach Formel (I) auch möglich, eine 100 %-Emittermaterial-Schicht aufzubauen. Für im Blauen emittierende erfindungsgemäße Emittermaterialien werden vorzugsweise UGH-Matrixmaterialien eingesetzt (vgl. M.E. Thompson et al., Chem. Mater. 2004, 16, 4743). Zur Erzeugung von mischfarbigem Licht bei der Verwendung von erfindungsgemäßen Verbindungen mit verschiedenen Metall-Zentralionen kann ebenfalls eine Co-Verdampfung angewendet werden.

Auf die Emitterschicht wird vorzugsweise eine Hole-Blocking-Schicht aufgetragen, welche Ohm'sche Verluste reduziert, die durch Lochströme zur Kathode entstehen könnten. Diese Hole-Blocking-Schicht ist vorzugsweise 10 bis 50 nm, insbesondere 15 bis 25 nm dick. Ein geeignetes Material hierfür ist beispielsweise BCP (4,7-Diphenyl-2,9-Dimethyl-Phenanthrolin, auch Bathocuproin genannt). Auf die Hole-Blocking-Schicht und zwischen diese Schicht und die Kathode wird vorzugsweise eine ETL-Schicht aus Elektronentransportmaterial (ETL = electron transport layer) aufgebracht. Vorzugsweise besteht diese Schicht aus aufdampfbarem Alq₃ mit einer Dicke von 10 bis 100 nm, insbesondere von 30 bis 50 nm. Zwischen die ETL-Schicht und die Kathode wird vorzugsweise eine Zwischenschicht aufgebracht, beispielsweise aus CsF oder LiF. Diese Zwischenschicht verringert die Elektroneninjektionsbarriere und schützt die ETL-Schicht. Diese Schicht wird in der Regel aufgedampft. Die Zwischenschicht ist vorzugsweise sehr dünn, insbesondere 0,5 bis 2 nm, mehr bevorzugt 0,8 bis 1,0 nm dick. Schließlich wird noch eine leitende Kathodenschicht aufgedampft, insbesondere mit einer Dicke von 50 bis 500 nm, mehr bevorzugt von 100 bis 250 nm. Die Kathodenschicht besteht vorzugsweise aus Al, Mg/Ag (insbesondere im Verhältnis 10:1) oder anderen Metallen. An den beschriebenen OLED-Aufbau für einen sublimierbaren erfindungsgemäßen Emitter werden vorzugsweise Spannungen zwischen 3 und 15 V angelegt.

Das OLED-Device kann auch teils nasschemisch gefertigt werden, und zwar zum Beispiel gemäß folgendem Aufbau: Glassubstrat, durchsichtige ITO-Schicht (aus Indium-Zinn-Oxid), z.B. PEDOT/PSS (z.B. 40 nm), 100 % erfindungsgemäßer Komplex nach Formel (I) (z.B. 10 bis 80 nm) oder Komplexe nach Formel (I) bzw. Formel (II) eindotiert (z.B. 1 %, insbesondere 4 % bis 10 %) in eine geeignete Matrix (z.B. 40 nm), aufgedampftes Alq₃ (z.B. 40 nm), aufgedampfte LiF oder CsF Schutzschicht (z.B. 0,8 nm), aufgedampfte Metallkathode Al oder Ag oder Mg/Ag (z.B. 200 nm).

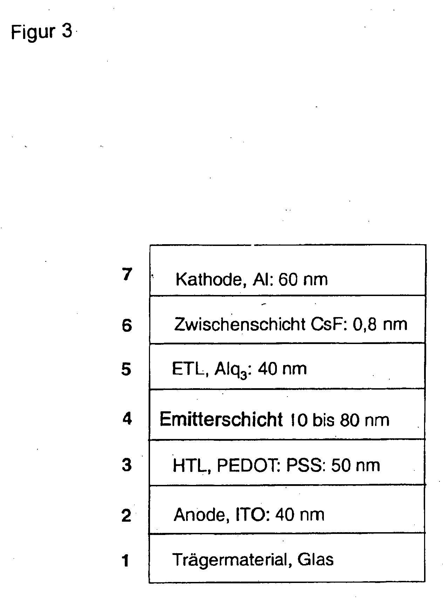

Besonders bevorzugt weist ein OLED-Aufbau für einen löslichen, erfindungsgemäßen Emitter die im Folgenden beschriebene und in Figur 3 dargestellte Struktur auf, umfasst aber wenigstens eine, mehr bevorzugt wenigstens zwei und am meisten bevorzugt alle der nachfolgend genannten Schichten.

Die Vorrichtung wird vorzugsweise auf ein Trägermaterial aufgebracht, insbesondere auf Glas oder ein anderes festes oder flexibles durchsichtiges Material. Auf das Trägermaterial wird eine Anode aufgebracht, beispielsweise eine Indium-Zinn-Oxid-Anode. Die Schichtdicke der Anode beträgt vorzugsweise 10 nm bis 100 nm, insbesondere 30 bis 50 nm. Auf die Anode und zwischen Anode und Emitterschicht wird eine HTL-Schicht (hole transport layer) aus einem Lochleitermaterial aufgebracht, insbesondere aus einem Lochleitermaterial, welches wasserlöslich ist. Ein solches Lochleitermaterial ist beispielsweise PEDOT/PSS (Polyethylendioxythiophen/Polystyrolsulfonsäure). Die Schichtdicke der HTL-Schicht beträgt vorzugsweise 10 bis 100 nm, insbesondere 40 bis 60 nm. Als Nächstes wird die Emitterschicht (EML) aufgebracht, welche einen erfindungsgemäßen löslichen Emitter enthält. Das Material kann in einem Lösungsmittel, beispielsweise in Aceton, Dichlormethan oder Acetonitril, gelöst werden. Dadurch kann ein Auflösen der darunterliegenden PEDOT/PSS-Schicht vermieden werden. Das erfindungsgemäße Emittermaterial kann für Komplexe der Formel (I) und Formel (II) in geringer Konzentration, z.B. 2 bis 10 Gew.-%, aber für Komplexe nach Formel (I) auch in höherer Konzentration oder als 100 %-Schicht eingesetzt werden. Es ist auch möglich, das Emittermaterial hoch- oder mitteldotiert in einer geeigneten Polymerschicht (z.B. PVK = Polyvinylcarbazol) aufzubringen. Die Dotierungskonzentration ist für den Fall der Ausnutzung der Metall-Metall-Wechselwirkung unter Verwendung der Komplexe der Formel (I) vorzugsweise so hoch, dass eine Dimer-, Trimer- oder Oligomer-Bildung des Emitters stattfinden kann.

Auf die Emitterschicht wird vorzugsweise eine Schicht aus Elektronentransportmaterial aufgebracht, insbesondere mit einer Schichtdicke von 10 bis 80 nm, mehr bevorzugt von 30 bis 50 nm. Ein geeignetes Material für die Elektronentransportmaterialschicht ist beispielsweise Alq₃, welches aufdampfbar ist. Als nächstes wird vorzugsweise eine dünne Zwischenschicht aufgebracht, welche die Elektroneninjektionsbarriere verringert und die ETL-Schicht schützt. Diese Schicht weist vorzugsweise eine Dicke zwischen 0,5 und 2 nm, insbesondere zwischen 0,5 und 1,0 nm auf und besteht vorzugsweise aus CsF oder LiF. Diese Schicht wird in der Regel aufgedampft. Für einen weiter vereinfachten OLED-Aufbau können gegebenenfalls der ETL- und/oder die Zwischenschicht entfallen.

Schließlich wird eine leitende Kathodenschicht aufgebracht, insbesondere aufgedampft. Die Kathodenschicht besteht vorzugsweise aus einem Metall, insbesondere aus Al oder Mg/Ag (insbesondere im Verhältnis 10:1).

An die Vorrichtung werden vorzugsweise Spannungen von 3 bis 15 V angelegt.

Erfindungswesentlich ist, dass die Licht emittierende Vorrichtung als Emitter wenigstens einen M-Oxazol-Komplex der Formel (I) oder (II) enthält, insbesondere einen Komplex, in dem X = O.

Erfindungsgemäß wurde festgestellt, dass Verbindungen der Formel (I) oder (II) hervorragend als Emitter-Moleküle für Licht emittierende Vorrichtungen und insbesondere für organische Licht emittierende Vorrichtungen (OLEDs) geeignet sind. Die erfindungsgemäßen Verbindungen sind insbesondere für den Einsatz in lichterzeugenden Systemen, wie zum Beispiel Displays oder Beleuchtungen, hervorragend geeignet.

Durch die Verwendung von M-Oxazol-Komplexen der Formel (I) oder (II) als Emittermaterialien in OLEDs ergibt sich eine Reihe von Vorteilen. So können beispielsweise Emitterschichten durch Sublimation hergestellt werden. Wegen der Dreibindigkeit der Oxazolliganden an das Metall sind die Emitterkomplexe besonders stabil, und zwar auch unter den relativ harten Sublimationsbedingungen, die für die technologische Fertigung erforderlich sind. Im Fall einer Verwendung von 100 % bzw. hochkonzentrierten Emitterschichten mit erfindungsgemäßen Materialien nach Formel (I) können bei der Fertigung der Vorrichtungen keine Konzentrationsschwankungen auftreten. Weiterhin ist es möglich, den Emitter in kristallinen Schichten bereitzustellen oder in Oligomerschichten. In diesen Systemen, insbesondere in kristallinen Schichten aus Komplexen der Formel (I), sind die Ladungsträger-Mobilitäten wesentlich höher als in amorphen Schichten. Weiterhin können mit den erfindungsgemäßen Emittermolekülen bei hohen Stromdichten hohe Leuchtdichten erzielt werden. Zudem kann auch bei hohen Stromdichten eine relativ hohe Effizienz (Quantenwirkungsgrad) erzielt werden. Die Komplexe der Formeln (I) und (II) können auch gelöst in geeigneten Matrizen in kleiner Dotierung (z.B. 2 - 10 %) erfindungsgemäß eingesetzt werden.

Die erfindungsgemäßen Komplexe der Formel (I) können in einer Ausführungsform der Erfindung vorteilhafterweise in hoher Konzentration in der Emitterschicht eingesetzt werden. Der Anteil an Komplexen der Formel (I) in der Emitterschicht beträgt dabei vorzugsweise mehr als 80 Gew.-%, insbesondere mehr als 90 Gew.-%, noch mehr bevorzugt mehr als 95 Gew.-%, und insbesondere 100 Gew.-%, bezogen auf das Gesamtgewicht der Emitterschicht. Bei solch hohen Konzentrationen entstehen Oligomere, die dann als Oligomer-Emitter wirken. Die Übergänge, die zur Emission führen, basieren dabei auf Metall-Metall-Wechselwirkungen zwischen den einzelnen Metallatomen der Komplexe in den Oligomeren. Je nach Dotierungsmenge und in Abhängigkeit der Reste R weisen die erfindungsgemäßen Verbindungen der Formel (I) in solchen Emitterschichten unterschiedliche M-M-Abstände auf, wodurch sich die Emissionsfarben über weite Bereiche ändern lassen.

In einer weiteren bevorzugten Ausführungsform werden die Komplexe der Formel (I) in mittleren Konzentrationen in der Emitterschicht eingesetzt, so dass nebeneinander Monomere und Oligomere vorliegen. Dadurch ist es möglich, eine mischfarbige Emission, z.B. weiß, zu erzielen. Der Anteil an Komplexen der Formel (I) beträgt hierbei in der Emitterschicht vorzugsweise mehr als 10 Gew.-%, insbesondere mehr als 20 Gew.-%, noch mehr bevorzugt mehr als 30 Gew.-%, und insbesondere mehr als 40 Gew.-% und bis zu 80 Gew.-%, insbesondere bis zu 70 Gew.-%, mehr bevorzugt bis zu 60 Gew.-%, jeweils bezogen auf das Gesamtgewicht der Emitterschicht.

In einer weiteren bevorzugten Ausführungsform der Erfindung werden Komplexe der Formel (I) oder/und der Formel (II) in geringer Konzentration in der Emitterschicht eingesetzt, wodurch eine Monomer-Emission in der OLED Vorrichtung erzielt wird. Die Komplexe der Formel (I) oder/und (II) liegen dabei in der Emitterschicht insbesondere mit mehr als 2 Gew.-%, insbesondere mehr als 4 Gew.-% und bis zu 10 Gew.-%, insbesondere bis zu 8 Gew.-%, bezogen auf das Gesamtgewicht der Emitterschicht, vor.

In einer weiteren bevorzugten Ausführungsform werden erfindungsgemäß in der Licht emittierenden Vorrichtung wenigstens zwei verschiedene Komplexe der Formel (I) oder (II) eingesetzt. Durch solche Emitterschichten mit mehreren Komplexen kann insbesondere mischfarbiges Licht erhalten werden. Bevorzugt umfasst die Emitterschicht wenigstens einen Komplex der Formel (I) mit M = Pt und mindestens einen Komplex der Formel (I) mit M = Pd. Besonders bevorzugt werden Emitterschichten, welche eine hohe Konzentration an Verbindungen mit M = Pd(II) aufweisen, mit Komplexen der Formel (I) mit M = Pt(II) mit geringer Konzentration dotiert. Der Einbau von Pt-Komplexen in Pd-Komplex-Emitterschichten, insbesondere Emitterschichten, in welchen Pd-Komplex-Oligomere vorliegen, führt zu mischfarbigem Licht der OLED Vorrichtungen.

In einer bevorzugten Ausführungsform weist die Emitterschicht Komplexe der Formel (I) und der Formel (II) in einer Konzentration von größer 1 Gew.-%, bezogen auf das Gesamtgewicht der Emitterschicht, insbesondere größer 2 Gew.-%, mehr bevorzugt größer 5 Gew.-% und bis zu 10 Gew.-%, insbesondere bis zu 8 Gew.-% auf. Es ist aber auch möglich, Emitterschichten bereitzustellen, die nahezu vollständig Komplexe der Formel (I) enthalten und insbesondere > 80 Gew.-% und am meisten bevorzugt > 90 Gew.-%, insbesondere > 95 Gew.-%, mehr bevorzugt > 99 Gew.-%. In einer weiteren Ausführungsform besteht die Emitterschicht vollständig, also zu 100 % aus Komplexen der Formel (I). Bei Einsatz der erfindungsgemäßen Komplexe in hoher Konzentration in der Emitterschicht bilden sich kristalline Schichten oder Stapel der Komplexe mit relativ kurzen Metall-Metall-Abständen aus. In diesen Stapeln treten starke elektronische Wechselwirkungen auf. Die Emissionswellenlänge wird dabei durch den MM-Abstand bestimmt. Die Verwendung von hochkonzentrierten Emitterschichten und insbesondere von kristallinen oder quasi-kristallinen Schichten bietet weitere Vorteile. Insbesondere treten bei der Fertigung keine Konzentrationsschwankungen auf bzw. wirken sich diese in hochkonzentrierten Systemen nur geringfügig aus. Weiterhin sind bei der Bildung von kristallinen Schichten die Ladungsträgermobilitäten, also die Elektronen- bzw. Loch-Beweglichkeiten deutlich höher als in amorphen Schichten. Weiterhin lässt sich mit derart konzentrierten Emitterschichten bei hohen Stromdichten eine hohe Leuchtdichte erzielen und eine hohe Effizienz, also einen hohen Quantenwirkungsgrad, erreichen.

Durch die hohe Ladungsträger-Beweglichkeit von Oligomeren bzw. kristallinen oder quasi-kristallinen Schichten aus Komplexen der Formel (I) kann insbesondere die Effizienz und die Langlebigkeit von OLED Vorrichtungen erhöht werden.

Die vorliegende Erfindung liefert u.a. die folgenden Vorteile:
- Völlig neue Molekülstrukturen mit dreizähniger Ligand-Metall-Bindung.
- Effizienter und heller Emitter durch Einschränkung der Molekülflexibilität.
- Durch Substitutionen Steuerung der Emissionsfarben vom Grünen bis ins Rote.
- Hohe thermische Stabilität.
- Gute Sublimierbarkeit und damit gute Eignung bei einem technischen Einsatz unter Verwendung der Methode der Vakuum-Deposition (vacuum vapor deposition).
- Hohe Langzeitstabilität.
- Hohe chemische Stabilität gegenüber Sauerstoff und Wasser.
- Extrem hohe chemische Variabilität.
- Gute Löslichkeit und damit gut geeignet zum Dotieren für Spin-Coating oder Inkjet-Printing-Verfahren in unterschiedliche Polymer-Matrix-Materialien (guter Einbau in die Emitterschicht).
- Gute Eignung zur chemischen Verknüpfung mit Polymeren, Funktionalisierung von Polymeren für Verwendung beim Spin-Coating, Inkjet-Printing usw.

Die erfindungsgemäß als Emitter eingesetzten Komplexe lassen sich auf einfache Weise (durch Wahl von geeigneten Matrixmaterialien) sowie besonders durch die Auswahl von elektronenziehenden bzw. -schiebenden Substituenten im Wellenlängenbereich abstimmen.

Bevorzugt werden Verbindungen eingesetzt, die bei einer Temperatur von > 20 °C und bei Temperaturen von besonders bevorzugt über 100 °C eine Emission zeigen.

Die Erfindung betrifft weiterhin die Verwendung einer Verbindung der Formel (I) oder (II), wie hierin definiert, als Emitter einer Licht emittierenden Vorrichtung, insbesondere in einer organischen Licht emittierenden Vorrichtung.

Die Erfindung wird durch die beigefügten Figuren und die nachfolgenden Beispiele weiter erläutert.
**Figur 1** zeigt ein Beispiel einer mittels Vakuum-Sublimationstechnik erstellbaren OLED-Vorrichtung mit erfindungsgemäßen Komplexen.
**Figur 2** zeigt ein Beispiel für eine differenzierte hocheffiziente OLED-Vorrichtung mit erfindungsgemäßen sublimierbaren Emittermaterialien.
**Figur 3** zeigt ein Beispiel für eine OLED-Vorrichtung für erfindungsgemäße Emitter, die nass-chemisch aufgetragen werden sollen. Die Schichtdickenangaben gelten als Beispielswerte.

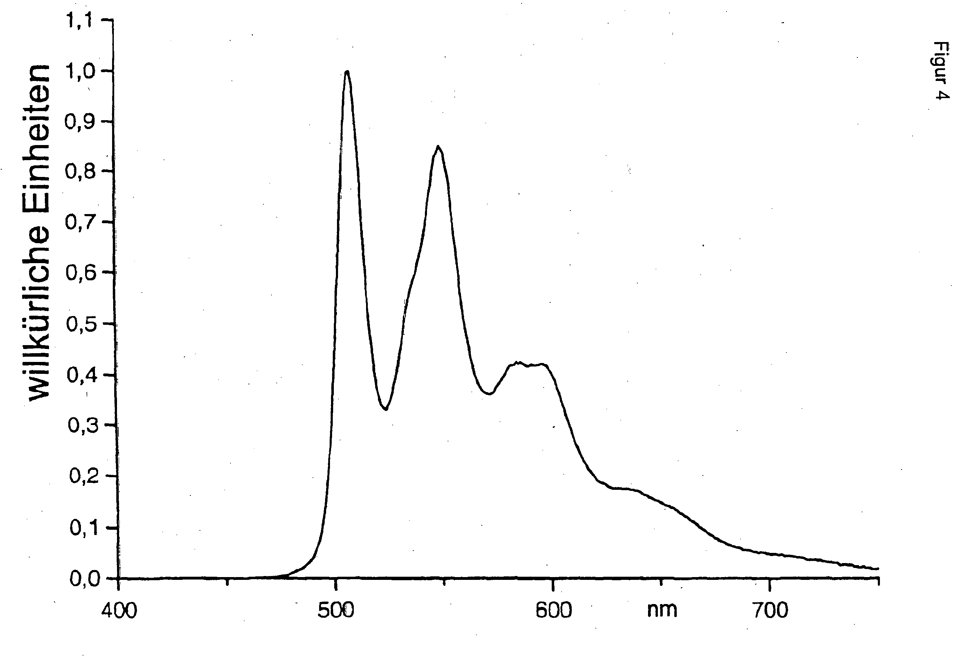

**Figur 4** zeigt das Emissionsspektrum vom Platinkomplex A. Die Bedingungen waren wie folgt: Anregung: 390,4 nm, Slits: 3,5/3,5 1,0 nm 0,3 s; Lösung in CHCl₃; Temperatur: 300 K; 20 min Argon gesättigt; Filter: KV 450.

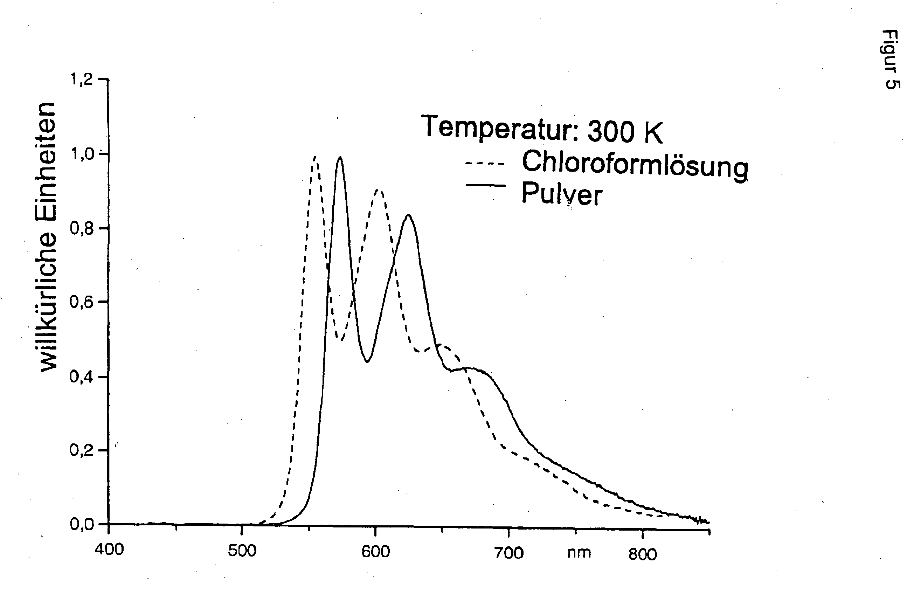

**Figur 5** zeigt das Lumineszenzspektrum vom Platinkomplex B gemessen in CHCl₃ (λₑₓ = 415 nm) bzw. als Feststoff (λₑₓ = 390 nm).

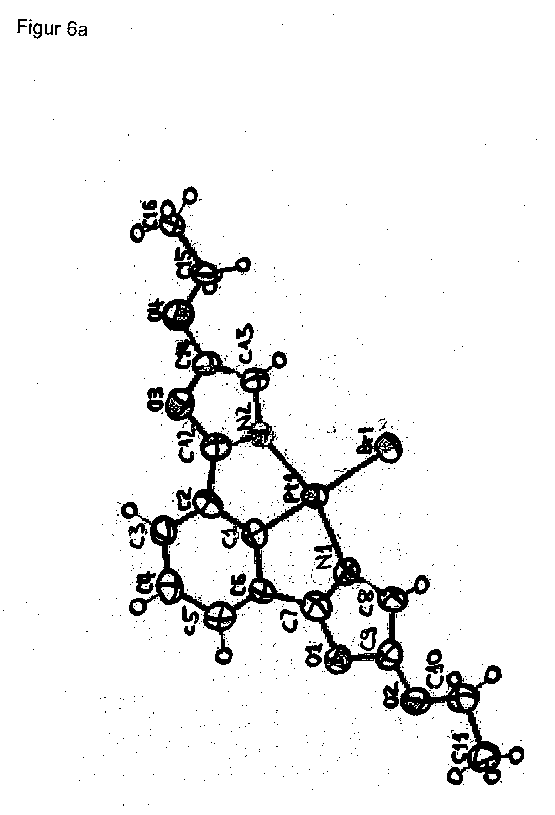

**Figur 6a** zeigt die Struktur vom Platinkomplex C.

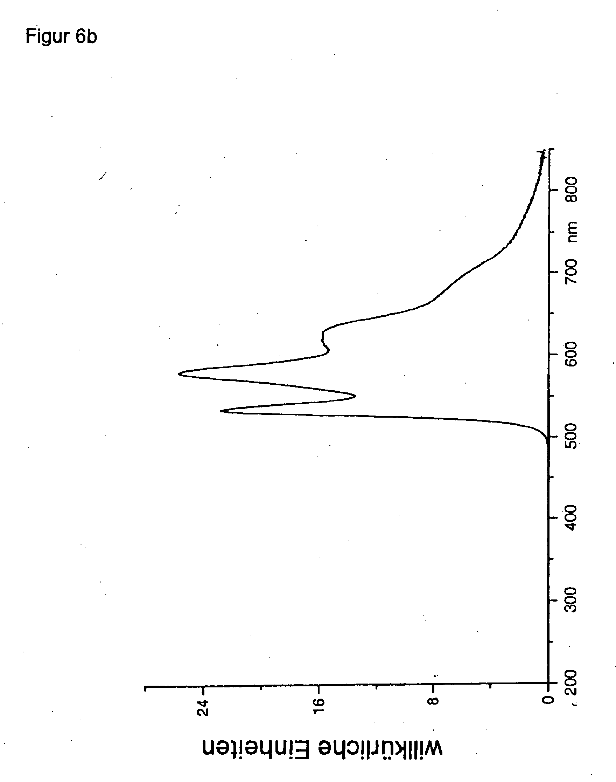

**Figur 6b** zeigt das Emissionsspektrum vom Platinkomplex C. Die Bedingungen waren wie folgt: Anregung: 370 nm; 0,5 nm, 25/2,5 0,3; Lösung in EtOH, 300 K; Filter: WG 420.

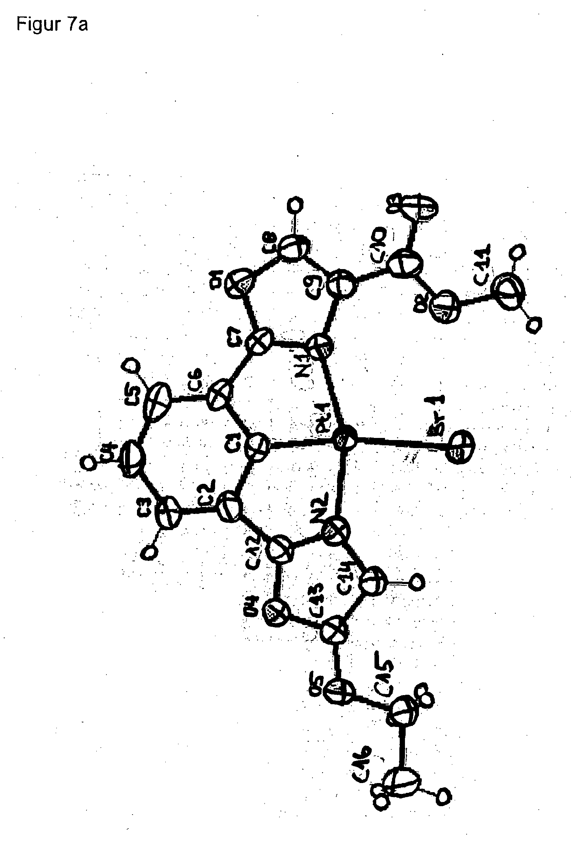

**Figur 7a** zeigt die Struktur vom Platinkomplex D.

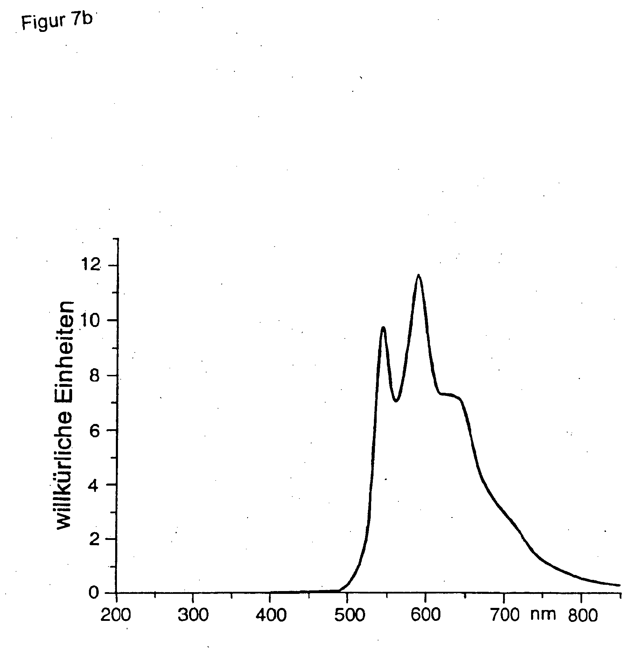

**Figur 7b** zeigt das Emissionsspektrum vom Platinkomplex D. DieBedingungen waren wie folgt: Anregung: 370 nm; 0,5 nm, 2,5/2,5 0,3; Lösung in EtOH, 300 K; Filter: WG 420.

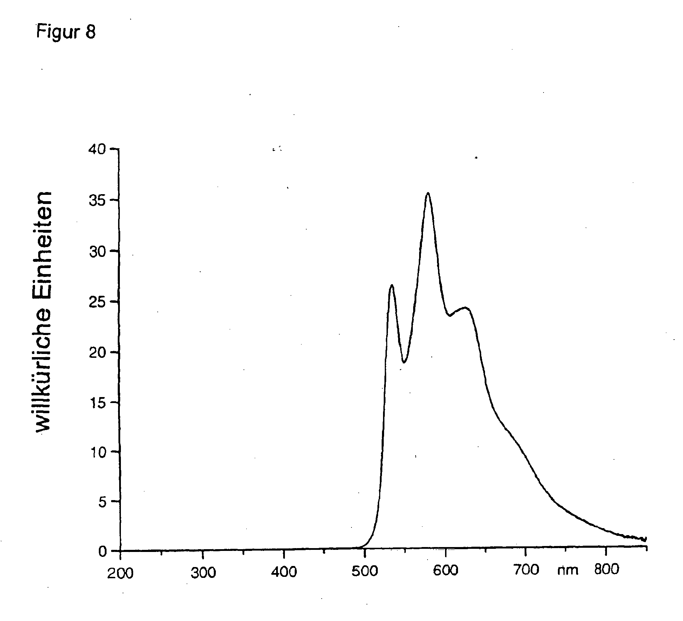

**Figur 8** zeigt das Emissionsspektrum vom Platinkomplex E. Die Bedingungen waren wie folgt: Anregung: 370 nm; 0,5 nm, 3/3 0,3; Lösung in EtOH, 300 K; Filter: WG 420; 20 min Ar gesättigt.

### Beispiele

### 1. Platinkomplex A

### 5,5'-Di-tert-butyl-2,2'-m-phenylene-bis-oxazole-2-platinum(II) bromid (A)

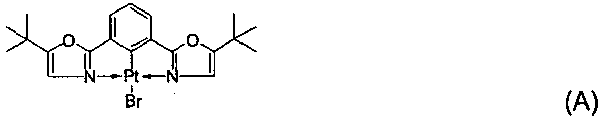

Zu einer Lösung von 2-Brom-1,3-di[2-(5-tert-butyl-oxazolyl)]benzol (60 mg, 0,15 mmol, 1,0 Äq) in 3 ml THF wurde Pt₂(dipdba)₃ (161 mg, 0,12 mmol, 1,6 Äq "Pt") unter Distickstoffaatmosphäre zugegeben. Das Reaktionsgemisch wurde über Nacht (20 h) bei 60 °C gerührt. Das Gemisch wurde im Vakuum konzentriert, um das Rohmaterial zuerhalten. Durch Säulenchromatographie (SiO₂, 2 x 24 cm, Hexan/EtOAc 3:1) wurde A als oranger Feststoff (77 mg, 0,13 mmol, 86 %) erhalten.

R_{f} (SiO₂, Hexan/EtOAc 3:1) = 0.30 (UV); M.p. > 295 °C (decomp.); ¹H-NMR (300 MHz, CDCl₃): δ = 7.52 - 7.43 (m, 2H), 7.31 - 7.18 (m, 3H), 1.38 (s, 18H); ¹³C-NMR (75.5 MHz, CDCl₃): δ = 172.2, 162.0, 157.3, 128.0, 123.3, 123.0, 119.8, 32.2, 28.4; IR (KBr): 3140, 3060, 2970, 2870, 1590, 1520, 1460, 1430, 1395, 1365, 1320, 1280, 1210, 1150, 1130, 1110, 1025, 1005, 940, 815, 720, 680 cm⁻¹; MS (PI-FDMS): m/z (%) = 1117.1 (40) [2M⁺-Br], 598.4 (100) [M⁺]; C₂₀A₂₃BrN₂O₂Pt (598.39): berechnet C 40.14, H 3.87, N 4.68; gefunden C 40.34, H 3.90, N 4.74.

### 2. Platinkomplex B

### 5,5'-Diphenyl-2,2'-m-phenylene-bis-oxazole-2-platinum(II) bromid (B)

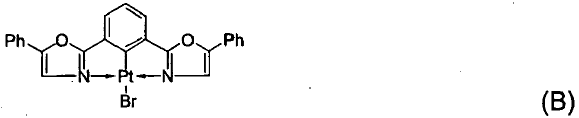

Zu einer Lösung von 2-Brom-1,3-di[2-(5-phenyl-oxazolyl)]benzol (35 mg, 0,079 mmol, 1,0 Äq) in 2 ml THF wurde Pt₂(dipdba)₃ (86 mg, 0,064 mmol, 1,6 Äq "Pt") unter Distickstoffatmosphäre zugegeben. Das Reaktionsgemisch wurde über Nacht (20 h) bei 60 °C gerührt. Es wurde eine Präzipitation und eine Gasentwicklung beobachtet. Nach der Reaktionszeit wurde CH₂Cl₂ dem Gemisch zugegeben. Durch Filtration wurde das Rohprodukt erhalten, welches aus CHCl₃/Et₂O umkristallisiert wurde, um 23 mg (0,036 mmol, 45 %) B als dunkelgelben Feststoff zu erhalten. Eine Zuordnung der Produkpeaks im ¹H-NMR-Spektrum war wiederum nicht möglich.

M.p. > 420 °C (decomp.); IR (KBr): 3160, 3070, 1590, 1570, 1515, 1490, 1455, 1400, 1365, 1325, 1252, 1210, 1160, 1135, 1010, 935, 810, 760, 725, 685 cm⁻¹; MS (PI-FDMS): m/z (%) = 1,197.6 (60) [2M⁺-Br], 638.6 (100) [M⁺]; C₂₄H₁₅BrN₂O₂Pt (638.37): berechnet C 45.16, H 2.37, N 4.39; gefunden C 44.48, H 2.55, N 4.25.

### 3. Platinkomplex C

### 2,6-Bis (5-ethoxy-oxazol-2-yl) phenyl-bromoplatin(II)

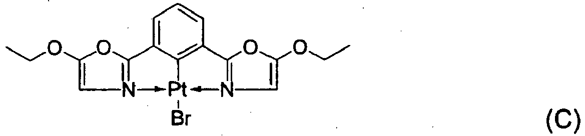

¹H NMR (300MHz, CDCl₃): δ = 7.31-7.25 (m, 2H), 7.16 (dd, *J* = 6.9, 8.5Hz, 1 H), 6.67(s, satellite *J_{PT-H}* = 9.2Hz, 2H), 4.26 (q, *J* = 7.0Hz, 4H), 1.50 (t, *J* = 7.1 Hz, 6H).
¹³C NMR (75MHz, CDCl₃): δ = 164.6, 159.0, 157.2, 128.1, 123.4, 121.4 (satellite *J_{Pt-C}* = 18.7Hz), 100.2 (satellite, *J_{Pt-C}* = 21.3Hz), 67.0, 14.5.
MS (FI-FDMS): m/z (%) = 1147.3 (41) [2M⁺], 1068.6 (60) [2M⁺-HBr], 573.9 (100) [M⁺].
Elementaranalyse: gefunden C 33.55, H 2.48, N 4.76, berechnet C 33.46, H 2.63, N 4.88

### 4. Platinkomplex D

### 2-(4-Methoxycarbonyl-oxazol-2-yl)-6-(5-ethoxy-oxazol-2-yl) phenyl-bromoplatin(II)

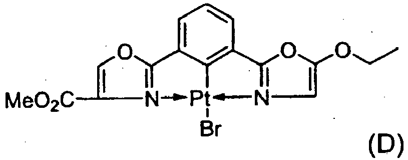

¹H NMR (300MHz, CD₂Cl₂): δ = 8.25(s, satellite *J_{Pt-H}* = 3.4Hz, 1 H), 7.45(d, *J* = 7.7 Hz, satellite *J_{Pt-H}* = 3.2Hz, 1 H), 7.33(d, *J* = 6.7 Hz, satellite *J_{Pt-H}* = 3.1 Hz, 1H), 7.16 (t, *J* = 6.9 Hz, 1 H), 6.82(s, satellite *J_{Pt-H}* = 10.5Hz, 1 H), 4.30 (q, *J* = 7.0Hz, 4H), 1.50(t, *J* = 7.1 Hz, 6H).
¹³C NMR (75MHz, CD₂Cl₂): δ = 175.4, 164.1, 159.5, 159.1, 156.6, 142.2 (satellite *J_{Pt-C}* = 15.0Hz), 134.2, 129.0, 126.8, 123.9, 123.6, 123.3, 99.9 (satellite *J_{Pt-C}* = 22.8Hz), 70.5, 53.4, 14.7.
MS (FI-FDMS): m/z (%) = 1176.0 (16) [2M⁺], 588.3 (100) [M⁺]. Elementaranalyse: gefunden C 32.78, H 2.18, N 4.74, berechnet C 32.67, H 2.23, N 4.76.

### 5. Platinkomplex E

### (3-Oxo-3-ethoxypropynyl)-[2,6-bis(5-ethoxy-oxazol-2-yl)phenyl]-platin(II)

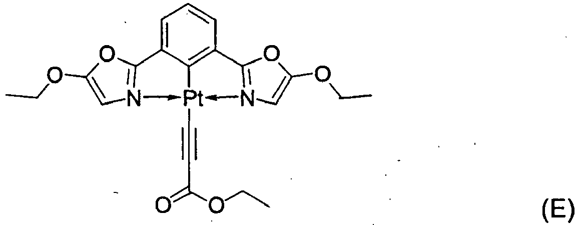

¹H NMR (300MHz, CDCl₃): δ = 7.35 (dd, *J* =7.4, 8.0Hz, 2H), 7.16 (dd, *J* = 7.1, 8.2Hz, 1H), 6.62(s, satellite *J_{Pt-H}* = 9.6Hz, 2H), 4.26 (q, *J* = 7.1 Hz, 4H), 4.23 (q, *J* = 7.2Hz, 2H),1.50(t, *J* = 7.1 Hz, 6H), 1.33(t, *J* = 7.1 Hz, 3H).
¹³C NMR (75MHz, CDCl₃): δ = 172.2, 166.7, 158.9, 154.4, 129.8, 129.4, 124.2, 121.3, 121.2, 102.4(satellite *J_{Pt-C}* = 25.4Hz), 69.7, 60.7, 14.5, 14.4. LR MS (FI-FDMS): m/z = 591.1 [M⁺].

### 6. Platinkomplex F

### (3-Hydroxylpropynyl)-[2,6-bis (5-ethoxy-oxazol-2-yl) phenyl]-platin(II)

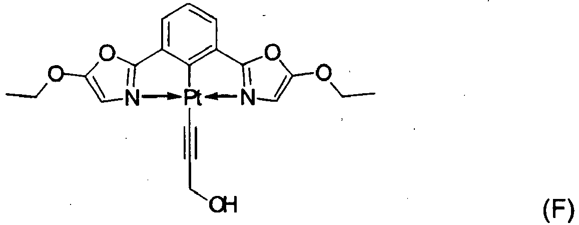

¹H NMR (300MHz, CDCl₃): δ = 7.37 (d, *J* = 7.7Hz, 2H), 7.16 (dd, *J* = 7.4, 8.0Hz, 1H), 6.67(s, satellite *J_{Pt-H}* = 9.7Hz, 2H), 4.62(d, *J* = 5.2Hz, satellite *J_{Pt-H}* = 7.8Hz, 2H), 4.27 (q, *J* = 7.0Hz, 4H), 1.60 (br t, *J* = 5.8Hz, 1 H), 1.50(t, *J* = 7.1Hz, 6H).

### 7. Palladiumkomplex G

### 5,5'-Di-tert-butyl-2,2'-m-phenylene-bis-oxazole-2-palladium(II) bromid

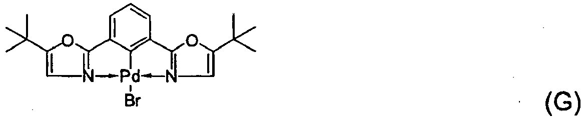

Pd(dba)₂ (223 mg, 0,388 mmol, 1.0 Äq) and 2-Brom-1,3-di-[2-(5-tert-butyl-oxazolyl)]benzol (157 mg, 0,389 mmol, 1,0 Äq) wurdenin trockenem Benzol gelöst (14 ml). Die Lösung wurde entgast (3 x Frieren-Pumpen-Auftauen-Zyklen) und unter Rückfluss erhitzt, bis die lila Farbe verblasst war (20 min). Das Reaktionsgemisch wurde unter Vakuum aufkonzentriert, wodurch das Rohmaterial erhalten wurde. Durch Säulenchromatographie (SiO₂, 3 x 20 cm, Hexane/EtOAc 3:1) wurde G (166 mg, 0,326 mmol, 84 %) als gelber Feststoff erhalten.

R_{f} (SiO₂, Hexane/EtOAc 3:1) = 0.18 (UV); M.p. > 290 °C (decomp.); ¹H-NMR (300 MHz, MeOH-d₄): δ = 7.25 - 7.20 (m, 2H), 7.12 (dd, 1 H, *J* = 8.5, 6.6 Hz), 6.89 (s, 2H), 1.42 (s, 18H); ¹³C-NMR (75.5 MHz, MeOH-d₄): δ = 168.6, 164.7, 162.7, 131.0, 126.0, 123.9, 121.0, 33.1, 29.0; IR (KBr): 3137, 3058, 2966, 2906, 2870, 2369, 1591, 1459, 1397, 1364, 1281, 1211, 1152, 1126, 1030, 1004, 946, 824, 724, 681 cm⁻¹; MS (PI-FDMS): m/z (%) = 939.5 (50) [2M⁺-Br], 510.4 (100) [M⁺], 429.4 (20) [M⁺-Br]; C₂₀H₂₃BrN₂O₂Pd (509.73): berechnet C 47.13, H 4.55, N 5.50; gefunden C 46.99, H 4.68, N 5.44.

### 8. Palladiumkomplex H

### 5,5'-Diphenyl-2,2'-m-phenylene-bis-oxazole-2-palladium(II) bromid

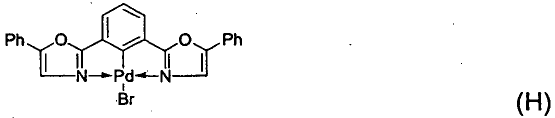

Pd(dba)₂ (115 mg, 0,200 mmol, 1,0 Äq) and 2-Brom-1,3-di[2-(5-phenyl-oxazolyl)]benzol (88,6 mg, 0,200 mmol, 1,0 Äq) wurden in trockenem Benzol (7 ml) gelöst. Die Lösung wurde entgast (3 x Frieren-Pumpen-Auftauen-Zyklen) und unter Rückfluss erhitzt, bis die lila Farbe blass war (20 min). Es wurde eine weiße bis graue Präzipitation beobachtet. Das Reaktionsgemisch wurde unter Vakuum aufkonzentriert. Die Zugabe von CH₂Cl₂ und Filtration des resultierenden Gemisches ergab einen grauen unlöslichen Filterkuchen (67 mg) und ein gelbes Filtrat. Das Filtrat wurde auf ein kleineres Volumen konzentriert und bei Zugabe von Et₂O begann das Produkt zu präzipitieren. Dieses Rohprodukt wurde aus CH₂Cl₂/Et₂O umkristallisiert, um 23 mg (0,042 mmol, 21 %) H als gelben lockeren Feststoff zu ergeben. Eine Zuordnung der Poduktpeaks im ¹H-NMR Spektrum war nicht möglich.

M.p. > 400 °C (decomp.); IR (KBr): 3143, 3051, 2373, 1589, 1524, 1487, 1452, 1389, 1318, 1250, 1201, 1155, 1130, 1004, 932, 813, 760, 726, 688 cm⁻¹; MS (PI-FDMS): m/z (%) = 1019.1 (25) [2M⁺-Br], 550.2 (100) [M⁺], 468.5 (60) [M⁺-Br], 444.3 (40) [M⁺-Pd], C₂₄H₁₅BrN₂O₂Pd (549.71): berechnet C 52.44, H 2.75, N 5.10; gefunden C 52.36, H 2.95, N 5.11.

## Patentansprüche

1. Komplex der Formel (I) oder (II) 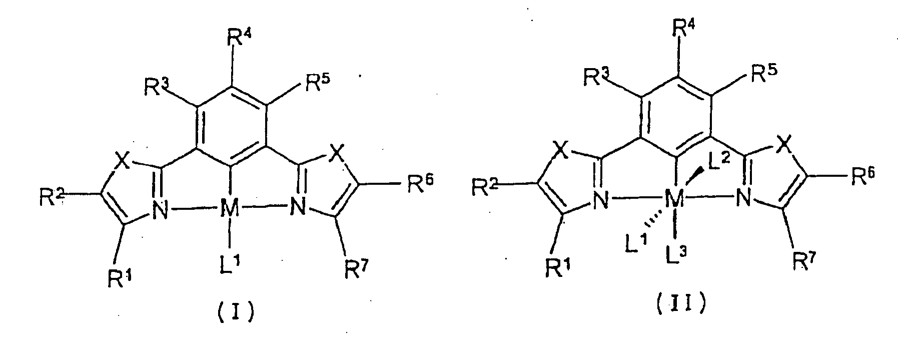 worin M ausgewählt ist aus Mo, Tc, Ru, Rh, Pd, Ag, W, Re, Os, Ir, Pt und Au, X ein Element der Gruppe 15 oder 16 des Periodensystems darstellt, R¹ bis R⁷ jeweils unabhängig voneinander Wasserstoff, Halogen, R', O-R' oder N-R'R" darstellen, worin R' eine Kohlenwasserstoffgruppe darstellt, welche gegebenenfalls Heteroatome enthalten kann und R" für H steht oder eine für R' angegebene Bedeutung besitzt, wobei zwei oder mehrere Gruppen R¹ bis R⁷ auch annelierte Ringsysteme bilden können und L¹, L² und L³ jeweils unabhängig voneinander einen negativ geladenen oder neutralen Liganden darstellen, wobei zwei oder mehr der Liganden L¹, L² und L³ miteinander verbunden sein können.

2. Komplex nach Anspruch 1 **dadurch gekennzeichnet, dass** M in Formel (I) oder (II) Pt(II), Pd(II), Ir(III), Ru(II) oder Os(II) darstellt.

3. Komplex nach Anspruch 1 oder 2, **dadurch gekennzeichnet, dass** X unabhängig Stickstoff, Sauerstoff oder Schwefel darstellt.

4. Komplex nach einem der vorhergehenden Ansprüche, **dadurch gekennzeichnet, dass** R¹ bis R⁷ oder R' oder R'' jeweils unabhängig voneinander Wasserstoff-, Alkyl-, Aryl-, Heteroaryl-, Alkenyl- oder Alkinylgruppen darstellen, welche gegebenenfalls substituiert sein können.

5. Komplex nach einem der vorhergehenden Ansprüche, **dadurch gekennzeichnet, dass** R¹ bis R⁷ oder R' oder R" jeweils unabhängig voneinander einen oder mehrere Substituenten aufweisen, ausgewählt aus Halogenen, insbesondere Fluor, und Alkylgruppen mit 1 bis 6, insbesondere 1 bis 4 C-Atomen.

6. Komplex nach einem der vorhergehenden Ansprüche, **dadurch gekennzeichnet, dass** L¹, L² und L³ jeweils unabhängig voneinander ausgewählt sind aus Halogen, insbesondere Chlorid oder Bromid, Pseudohalogen, insbesondere Thiocyanat, Cyanat oder Cyanid, und Liganden, die über ein Element der 16. Gruppe des Periodensystems, insbesondere Sauerstoff oder Schwefel, der 15. Gruppe des Periodensystems, insbesondere Stickstoff, Phosphor oder Arsen, oder der 14. Gruppe des Periodensystems, insbesondere Kohlenstoff an M gebunden sind.

7. Komplex nach einem der vorhergehenden Ansprüche, ausgewählt aus 5,5'-Di-tert-butyl-2,2 -m-phenylen-bis-oxazol-2-platinum(II)bromid, 5,5'-Diphenyl-2,2'-m-phenylen-bis-oxazol-2-platinum(II)bromid, 2,6-bis(5-ethoxy-oxazol-2-yl)phenyl-bromoplatinum(II), 2-(4-methodycarbonyl-oxazol-2-yl)-6-(5-ethoxy-oxazol-2-yl)phenylbromoplatinum(II), (3-Oxo-3-ethoxypropynyl)-[2,6-bis(5-ethoxy-oxazol-2-yl)phenyl]-platinum(II), (3-hydroxylpropynyl)-[2,6-bis(5-ethoxy-oxazol-2-yl)phenyl]-platinum(II), 5,5'-Di-tert-butyl-2,2'-m-phenylene-bis-oxazol-2-palladium(II)bromid, 5,5'-Diphenyl-2,2'-m-phenylen-bis-oxazol-2-palladium(II)bromid, sowie aus

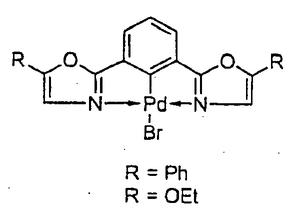

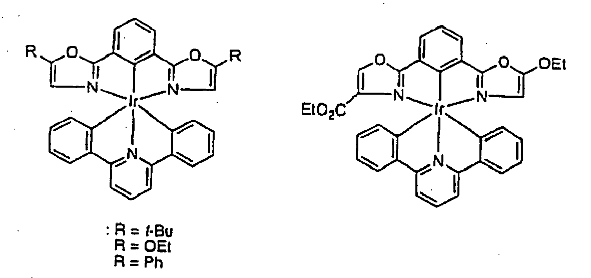

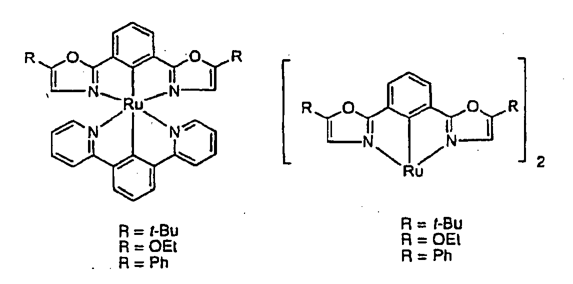

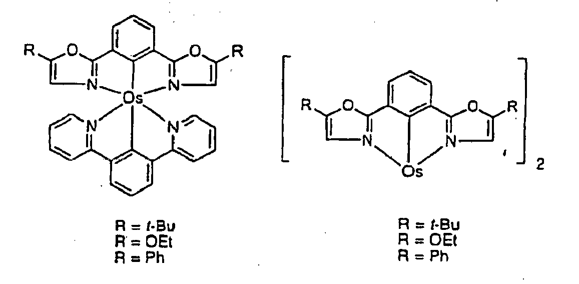

8. Ligand der Formel (III) 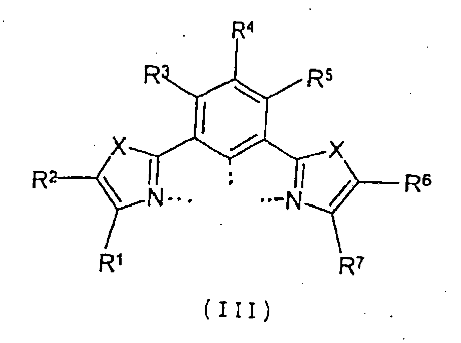 worin X ein Element der Gruppe 15 oder 16 des Periodensystems darstellt, R¹ bis R⁷ jeweils unabhängig voneinander Wasserstoff, Halogen, R', O-R' oder N-R'R" darstellen, worin R' eine Kohlenwasserstoffgruppe darstellt, welche gegebenenfalls Heteroatome enthalten kann und R" für H steht oder eine für R' angegebene Bedeutung besitzt, wobei zwei oder mehrere Gruppen R¹ bis R⁷ auch annelierte Ringsysteme bilden können.

9. Verbindung der Formel (IV) 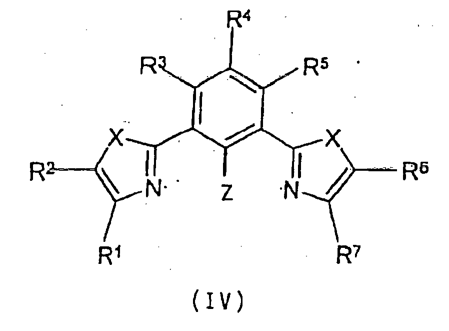 worin X ein Element der Gruppe 15 oder 16 des Periodensystems darstellt, R¹ bis R⁷ jeweils unabhängig voneinander Wasserstoff, Halogen, R', O-R' oder N-R'R" darstellen, worin R' eine Kohlenwasserstoffgruppe darstellt, welche gegebenenfalls Heteroatome enthalten kann und R" für H steht oder eine für R' angegebene Bedeutung besitzt, wobei zwei oder mehrere Gruppen R¹ bis R⁷ auch annelierte Ringsysteme bilden können und Z eine Abgangsgruppe, insbesondere H oder Halogen darstellt.

10. Verbindung nach Anspruch 9, ausgewählt aus

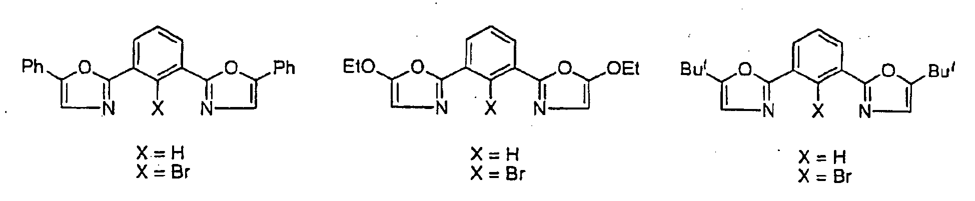

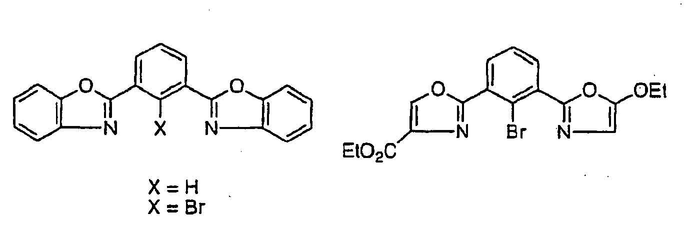

11. Licht emittierende Vorrichtung umfassend
(i) eine Anode,
(ii) eine Kathode und
(iii) eine Emitterschicht, angeordnet zwischen und in direktem oder indirektem Kontakt mit der Anode und der Kathode, umfassend wenigstens einen Komplex der Formel (I) oder (II) 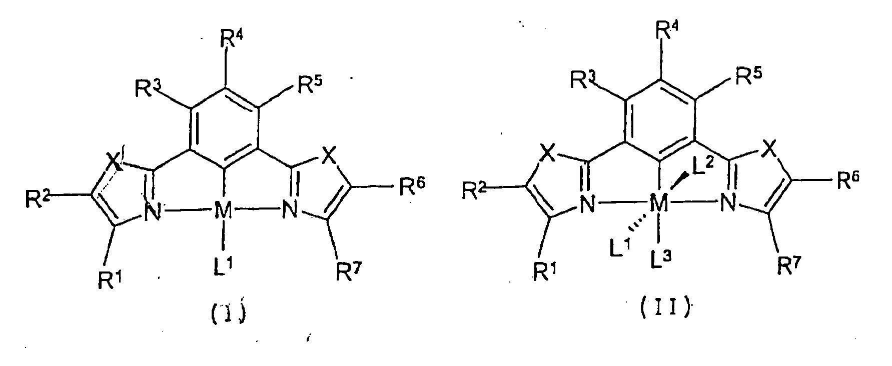 worin M ausgewählt ist aus Mo, Tc, Ru, Rh, Pd, Ag, W, Re, Os, Ir, Pt und Au, X ein Element der Gruppe 15 oder 16 des Periodensystems darstellt, R¹ bis R⁷ jeweils unabhängig voneinander Wasserstoff, Halogen, R', O-R' oder N-R'R" darstellen, worin R' eine Kohlenwasserstoffgruppe darstellt, welche gegebenenfalls Heteroatome enthalten kann und R'' für H steht oder eine für R' angegebene Bedeutung besitzt, wobei zwei oder mehrere Gruppen R¹ bis R⁷ auch annelierte Ringsysteme bilden können und L¹, L² und L³ jeweils unabhängig voneinander einen negativ geladenen oder neutralen Liganden darstellen, wobei zwei oder mehr der Liganden L¹, L² und L³ miteinander verbunden sein können.

12. Licht emittierende Vorrichtung nach Anspruch 11, **dadurch gekennzeichnet, dass** sie weiterhin eine Lochleiterschicht oder/und eine Elektronenleiterschicht umfasst.

13. Licht emittierende Vorrichtung nach einem der vorhergehenden Ansprüche 11 oder 12, **dadurch gekennzeichnet, dass** der in der Emitterschicht enthaltene Komplex ein Triplett-Emitter ist.

14. Licht emittierende Vorrichtung nach einem der vorhergehenden Ansprüche 11 bis 13, **dadurch gekennzeichnet, dass** M in einer Oxidationsstufe 0 bis +4 vorliegt.

15. Licht emittierende Vorrichtung nach einem der vorhergehenden Ansprüche 11 bis 14, **dadurch gekennzeichnet, dass** in der Emitterschicht Komplexe der Formel (I) oder/und (II) in einer Konzentration von 1 bis 100 Gew.-%, bezogen auf das Gesamtgewicht der Emitterschicht, enthalten sind.

16. Licht emittierende Vorrichtung nach einem der vorhergehenden Ansprüche 11 bis 15, **dadurch gekennzeichnet, dass** der Anteil an Komplexen der Formel (I) in der Emitterschicht mehr als 80 Gew.-%, insbesondere mehr als 90 Gew.-%, bezogen auf das Gesamtgewicht der Emitterschicht, beträgt.

17. Licht emittierende Vorrichtung nach Anspruch 16, **dadurch gekennzeichnet, dass** die Komplexe der Formel (I) in der Emitterschicht als Oligomere vorliegen.

18. Licht emittierende Vorrichtung nach einem der Ansprüche 11 bis 15, **dadurch gekennzeichnet, dass** der Anteil an Komplexen der Formel (I) in der Emitterschicht mehr als 10 Gew.-%, insbesondere mehr als 20 Gew.-% und bis zu 80 Gew.-%, insbesondere bis zu 70 Gew.-%, bezogen auf das Gesamtgewicht der Emitterschicht, beträgt.

19. Licht emittierende Vorrichtung nach einem der vorhergehenden Ansprüche, **dadurch gekennzeichnet, dass** sie als Emitter die Verbindung 5,5'-Di-tert-butyl-2,2'-m-phenylen-bis-oxazol-2-platinum(II)bromid, 5,5-Diphenyl-2,2-m-phenylen-bis-oxazol-2-platinum(II)bromid, 2,6-bis(5-ethoxy-oxazol-2-yl)phenyl-bromoplatinum(II), 2-(4-methodycarbonyl-oxazol-2-yl)-6-(5-ethoxy-oxazol-2-yl)phenyl-bromoplatinum(II), (3-Oxo-3-ethoxypropynyl)-[2,6-bis(5-ethoxy-oxazol-2-yl)phenyl]-platinum(II), (3-hydroxylpropynyl)-[2,6-bis(5-ethoxy-oxazol-2-yl)phenyl]-platinum(II), 5,5'-Di-tert-butyl-2,2'-m-phenylene-bis-oxazol-2-palladium(II)bromid, 5,5'-Diphenyl-2,2-m-phenylen-bis-oxazol-2-palladium(II)bromid, oder 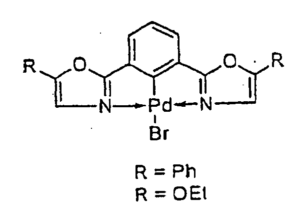 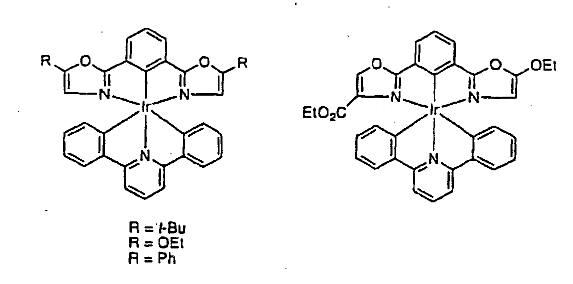 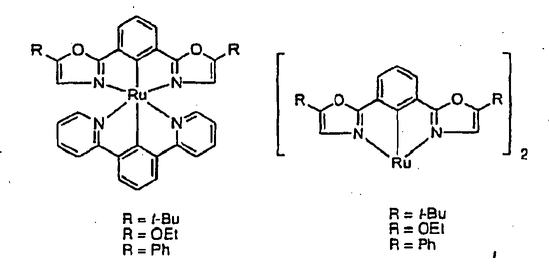 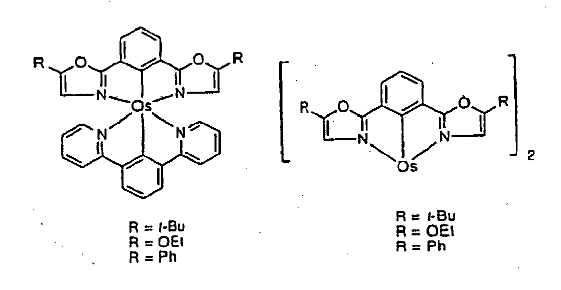 umfasst.

20. Licht emittierende Vorrichtung nach einem der vorhergehenden Ansprüche 11 bis 19, **dadurch gekennzeichnet, dass** sie wenigstens zwei verschiedene Komplexe der Formel (I) oder (II) umfasst.

21. Licht emittierende Vorrichtung nach einem der Ansprüche 11 bis 15, **dadurch gekennzeichnet, dass** in der Emitterschicht Komplexe der Formel (I) mit M = Pd(II) in einem Anteil von mehr als 80 Gew.-% und Komplexe der Formel (I) mit M = Pt(II) in einem Anteil von weniger als 10 Gew.-%, jeweils bezogen auf das Gesamtgewicht der Emitterschicht, vorliegen.

22. Licht emittierende Vorrichtung nach einem der vorhergehenden Ansprüche 11 bis 21, **dadurch gekennzeichnet, dass** sie als kristalline oder/und quasi-kristalline Schichten aus Komplexen der Formel (I) aufweist.

23. Licht emittierende Vorrichtung nach einem der vorhergehenden Ansprüche 11 bis 22, **dadurch gekennzeichnet, dass** es sich um ein OLED handelt.

24. Licht emittierende Vorrichtung nach einem der vorhergehenden Ansprüchen 11 bis 23, **dadurch gekennzeichnet, dass** es sich um ein Display oder/und eine Beleuchtungsvorrichtung handelt.

25. Verwendung eines Komplexes der Formel (I) oder (II) als Emitter in einer Licht emittierenden Vorrichtung.

26. Verfahren zur Herstellung einer Licht emittierenden Vorrichtung nach einem der Ansprüche 11 bis 24, **dadurch gekennzeichnet, dass** zumindest ein Komplex der Formel (I) oder (II) in der Emitterschicht mittels Vakuumsublimation eingebracht wird.

27. Verfahren zur Herstellung einer Licht emittierenden Vorrichtung nach einem der Ansprüche 11 bis 24, **dadurch gekennzeichnet, dass** zumindest der Komplex der Formel (I) oder (II) in der Emitterschicht nass-chemisch eingebracht wird.

## Claims

1. Complex of the formula (I) or (II) 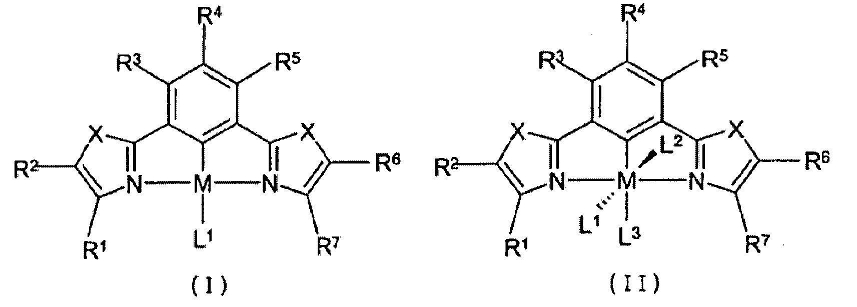 in which M is selected from Mo, Tc, Ru, Rh, Pd, Ag, W, Re, Os, Ir, Pt and Au, X represents an element from group 15 or 16 of the Periodic Table, R¹ to R⁷ each, independently of one another, represent hydrogen, halogen, R', O-R' or N-R'R", in which R' represents a hydrocarbon group, which may optionally contain heteroatoms, and R" stands for H or has a meaning indicated for R', where two or more groups R¹ to R⁷ may also form fused ring systems, and L¹, L² and L³ each, independently of one another, represent a negatively charged or neutral ligand, where two or more of the ligands L¹, L² and L³ may be connected to one another.

2. Complex according to Claim 1, **characterised in that** M in formula (I) or (II) represents Pt(II), Pd(II), Ir(III), Ru(II) or Os(II).

3. Complex according to Claim 1 or 2, **characterised in that** X independently represents nitrogen, oxygen or sulfur.

4. Complex according to one of the preceding claims, **characterised in that** R¹ to R⁷ or R' or R" each, independently of one another, represent hydrogen, alkyl, aryl, heteroaryl, alkenyl or alkynyl groups, which may optionally be substituted.

5. Complex according to one of the preceding claims, **characterised in that** R¹ to R⁷ or R' or R" each, independently of one another, have one or more substituents selected from halogens, in particular fluorine, and alkyl groups having 1 to 6, in particular 1 to 4 C atoms.

6. Complex according to one of the preceding claims, **characterised in that** L¹, L² and L³ are each selected, independently of one another, from halogen, in particular chloride or bromide, pseudohalogen, in particular thiocyanate, cyanate or cyanide, and ligands which are bonded to M via an element from group 16 of the Periodic Table, in particular oxygen or sulfur, group 15 of the Periodic Table, in particular nitrogen, phosphorus or arsenic, or group 14 of the Periodic Table, in particular carbon.

7. Complex according to one of the preceding claims, selected from 5,5'-di-tert-butyl-2,2'-m-phenylenebisoxazole-2-platinum(II) bromide, 5,5'-diphenyl-2,2'-m-phenylenebisoxazole-2-platinum(II) bromide, 2,6-bis(5-ethoxyoxazol-2-yl)phenylplatinum(II) bromide, 2-(4-methoxycarbonyloxazol-2-yl)-6-(5-ethoxyoxazol-2-yl)phenylplatinum(II) bromide, (3-oxo-3-ethoxypropynyl)-[2,6-bis(5-ethoxyoxazol-2-yl)phenyl]platinum(II), (3-hydroxylpropynyl)-[2,6-bis(5-ethoxyoxazol-2-yl)phenyl]platinum(II), 5,5'-di-tert-butyl-2,2'-m-phenylenebisoxazole-2-palladium(II) bromide, 5,5'-diphenyl-2,2'-m-phenylenebisoxazole-2-palladium(II) bromide, and from

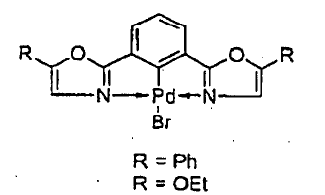

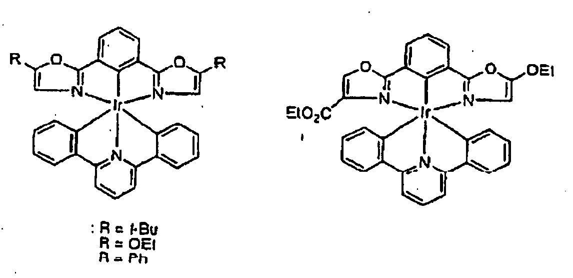

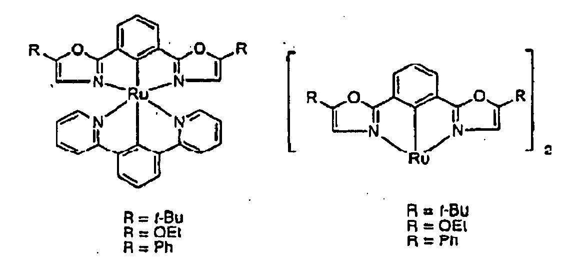

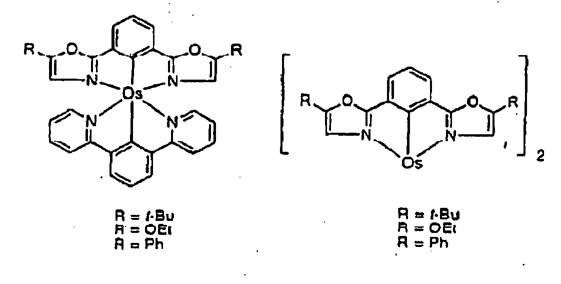

8. Ligand of the formula (III) 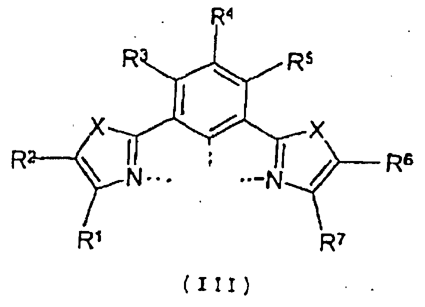 in which X represents an element from group 15 or 16 of the Periodic Table, R¹ to R⁷ each, independently of one another, represent hydrogen, halogen, R', O-R' or N-R'R", in which R' represents a hydrocarbon group, which may optionally contain heteroatoms, and R" stands for H or has a meaning indicated for R', where two or more groups R¹ to R⁷ may also form fused ring systems.

9. Compound of the formula (IV) 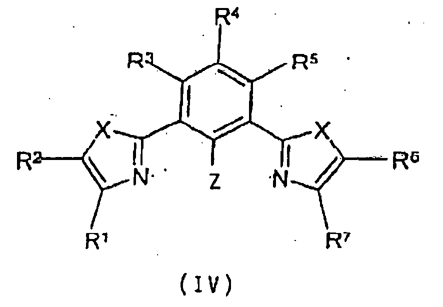 in which X represents an element from group 15 or 16 of the Periodic Table, R¹ to R⁷ each, independently of one another, represent hydrogen, halogen, R', O-R' or N-R'R", in which R' represents a hydrocarbon group, which may optionally contain heteroatoms, and R" stands for H or has a meaning indicated for R', where two or more groups R¹ to R⁷ may also form fused ring systems, and Z represents a leaving group, in particular H or halogen.

10. Compound according to Claim 9, selected from

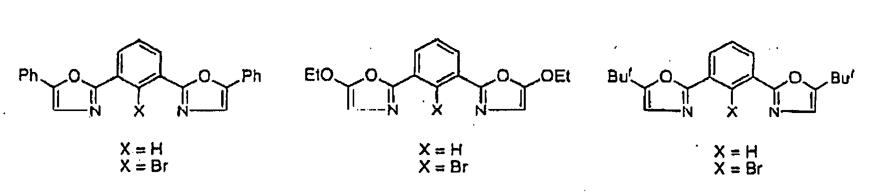

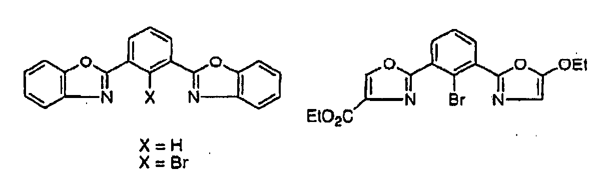

11. Light-emitting device comprising
(i) an anode,
(ii) a cathode and
(iii) an emitter layer, arranged between and in direct or indirect contact with the anode and the cathode, comprising at least one complex of the formula (I) or (II) 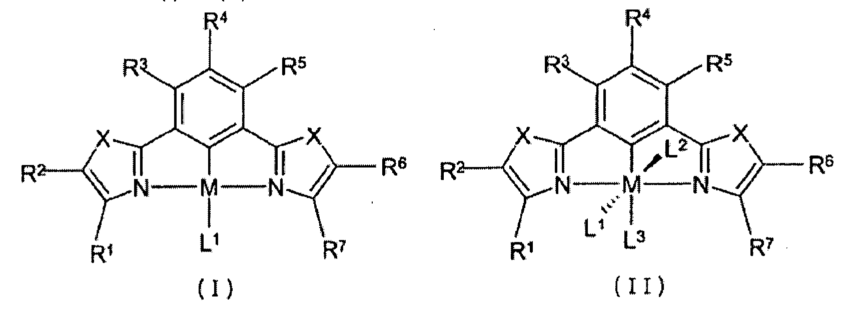 in which M is selected from Mo, Tc, Ru, Rh, Pd, Ag, W, Re, Os, Ir, Pt and Au, X represents an element from group 15 or 16 of the Periodic Table, R¹ to R⁷ each, independently of one another, represent hydrogen, halogen, R', O-R' or N-R'R", in which R' represents a hydrocarbon group, which may optionally contain heteroatoms, and R" stands for H or has a meaning indicated for R', where two or more groups R¹ to R⁷ may also form fused ring systems, and L¹, L² and L³ each, independently of one another, represent a negatively charged or neutral ligand, where two or more of the ligands L¹, L² and L³ may be connected to one another.

12. Light-emitting device according to Claim 11, **characterised in that** it furthermore comprises a hole-conductor layer or/and an electron-conductor layer.

13. Light-emitting device according to one of the preceding Claims 11 or 12, **characterised in that** the complex present in the emitter layer is a triplet emitter.

14. Light-emitting device according to one of the preceding Claims 11 to 13, **characterised in that** M is in an oxidation state 0 to +4.

15. Light-emitting device according to one of the preceding Claims 11 to 14, **characterised in that** complexes of the formula (I) or/and (II) are present in the emitter layer in a concentration of 1 to 100% by weight, based on the total weight of the emitter layer.

16. Light-emitting device according to one of the preceding Claims 11 to 15, **characterised in that** the proportion of complexes of the formula (I) in the emitter layer is greater than 80% by weight, in particular greater than 90% by weight, based on the total weight of the emitter layer.

17. Light-emitting device according to Claim 16, **characterised in that** the complexes of the formula (I) are present in the emitter layer as oligomers.

18. Light-emitting device according to one of Claims 11 to 15, **characterised in that** the proportion of complexes of the formula (I) in the emitter layer is greater than 10% by weight, in particular greater than 20% by weight and up to 80% by weight, in particular up to 70% by weight, based on the total weight of the emitter layer.

19. Light-emitting device according to one of the preceding claims, **characterised in that** it comprises, as emitter, the compound 5,5'-di-tert-butyl-2,2'-m-phenylene-bisoxazole-2-platinum(II) bromide, 5,5'-diphenyl-2,2'-m-phenylenebisoxazole-2-platinum(II) bromide, 2,6-bis(5-ethoxyoxazol-2-yl)phenylplatinum(II) bromide, 2-(4-methoxycarbonyloxazol-2-yl)-6-(5-ethoxyoxazol-2-yl)phenylplatinum(II) bromide, (3-oxo-3-ethoxypropynyl)-[2,6-bis(5-ethoxyoxazol-2-yl)phenyl]platinum(II), (3-hydroxylpropynyl)-[2,6-bis(5-ethoxyoxazol-2-yl)phenyl]platinum(II), 5,5'-di-tert-butyl-2,2'-m-phenylenebisoxazole-2-palladium(II) bromide, 5,5'-diphenyl-2,2'-m-phenylenebisoxazole-2-palladium(II) bromide, or

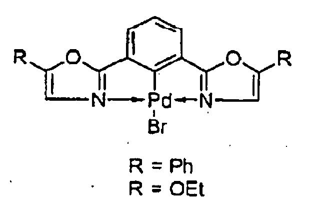

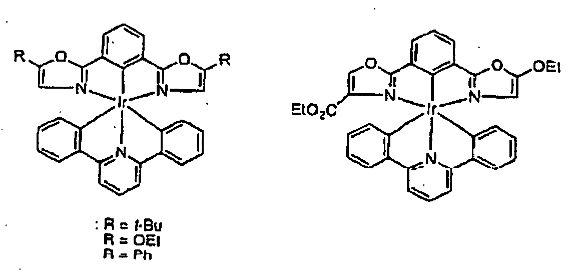

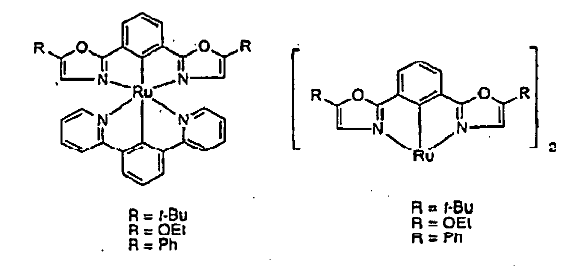

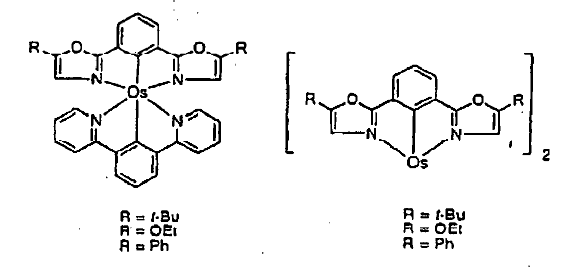

20. Light-emitting device according to one of the preceding Claims 11 to 19, **characterised in that** it comprises at least two different complexes of the formula (I) or (II).

21. Light-emitting device according to one of Claims 11 to 15, **characterised in that** complexes of the formula (I) where M = Pd(II) are present in the emitter layer in a proportion of greater than 80% by weight and complexes of the formula (I) where M = Pt(II) in a proportion of less than 10% by weight, in each case based on the total weight of the emitter layer.

22. Light-emitting device according to one of the preceding Claims 11 to 21, **characterised in that** it comprises crystalline or/and quasi-crystalline layers comprising complexes of the formula (I).

23. Light-emitting device according to one of the preceding Claims 11 to 22, **characterised in that** it is an OLED.

24. Light-emitting device according to one of the preceding Claims 11 to 23, **characterised in that** it is a display or/and a lighting device.

25. Use of a complex of the formula (I) or (II) as emitter in a light-emitting device.

26. Process for the production of a light-emitting device according to one of Claims 11 to 24, **characterised in that** at least one complex of the formula (I) or (II) is introduced in the emitter layer by means of vacuum sublimation.

27. Process for the production of a light-emitting device according to one of Claims 11 to 24, **characterised in that** at least the complex of the formula (I) or (II) is introduced in the emitter layer by wet-chemical methods.

## Revendications

1. Complexe de formule (I) ou (II) 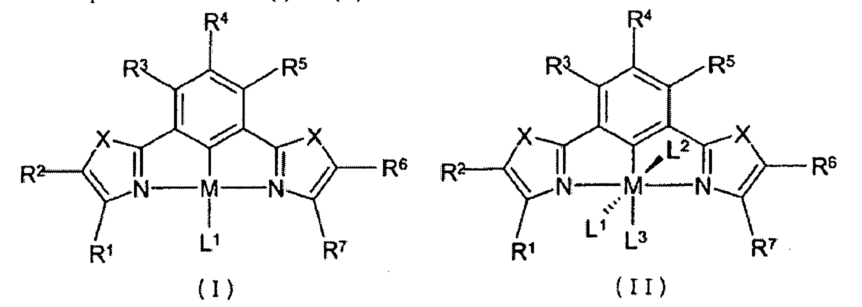 dans laquelle M est choisi parmi Mo, Tc, Ru, Rh, Pd, Ag, W, Re, Os, Ir, Pt et Au, X représente un élément du Groupe 15 ou 16 du Tableau Périodique, R¹ à R⁷ représentent chacun, indépendamment l'un de l'autre, hydrogène, halogène, R', O-R' ou N-R'R", où R' représente un groupement hydrocarboné, pouvant éventuellement contenir des hétéroatomes, et R" représente H ou revêt une signification indiquée pour R', où deux, ou plus, groupements R¹ à R⁷ peuvent également former des noyaux condensés, et L¹, L² et L³ représentent chacun, indépendamment les uns des autres, un ligand négativement chargé ou neutre, où deux, ou plus, parmi les ligands L¹, L² et L³ peuvent être reliés l'un à l'autre.

2. Complexe selon la revendication 1, **caractérisé en ce que** M dans la formule (I) ou (II) représente Pt(II), Pd(II), Ir(III), Ru(II) ou Os(II).

3. Complexe selon la revendication 1 ou 2, **caractérisé en ce que** X représente indépendamment azote, oxygène ou soufre.

4. Complexe selon l'une des revendications précédentes, **caractérisé en ce que** R¹ à R⁷ ou R' ou R" représentent chacun, indépendamment l'un de l'autre, hydrogène, des groupements alkyle, aryle, hétéroaryle, alcényle ou alcynyle, pouvant être éventuellement substitués.

5. Complexe selon l'une des revendications précédentes, **caractérisé en ce que** R¹ à R⁷ ou R' ou R" possèdent chacun, indépendamment les uns des autres, un ou plusieurs substituants choisis parmi des halogènes, en particulier fluor, et des groupements alkyle ayant de 1 à 6, en particulier de 1 à 4, atomes de C.

6. Complexe selon l'une des revendications précédentes, **caractérisé en ce que** L¹, L² et L³ sont choisis chacun, indépendamment les uns des autres, parmi halogène, en particulier chlorure ou bromure, pseudo-halogène, en particulier thiocyanate, cyanate ou cyanure, et des ligands qui sont liés à M via un élément du Groupe 16 du Tableau Périodique, en particulier oxygène ou soufre, du Groupe 15 du Tableau Périodique, en particulier azote, phosphore ou arsenic, ou du Groupe 14 du Tableau Périodique, en particulier carbone.

7. Complexe selon l'une des revendications précédentes, choisi parmi
le bromure de 5,5'-di-tertio-butyl-2,2'-m-phénylènebisoxazole-2-platine(II),
le bromure de 5,5'-diphényl-2,2'-m-phénylènebisoxazole-2-platine(II),
le bromure de 2,6-bis(5-éthoxyoxazol-2-yl)phénylplatine(II),
le bromure de 2-(4-méthoxycarbonyloxazol-2-yl)-6-(5-éthoxyoxazol-2-yl)-phénylplatine(II),
le (3-oxo-3-éthoxypropynyl)-[2,6-bis(5-éthoxyoxazol-2-yl)phényl]platine(II),
le (3-hydroxypropynyl)-[2,6-bis(5-éthoxyoxazol-2-yl)phényl]platine(II),
le bromure de 5,5'-di-tertio-butyl-2,2'-m-phénylènebisoxazole-2-palladium(II),
le bromure de 5,5'-diphényl-2,2'-m-phénylènebisoxazole-2-palladium(II), et parmi

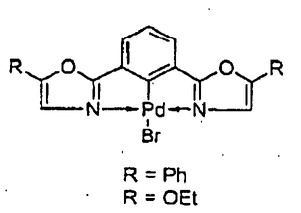

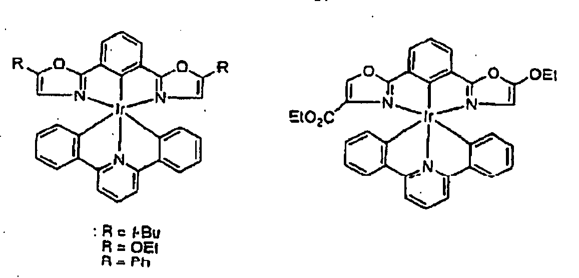

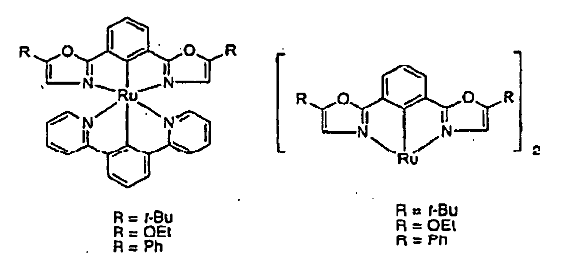

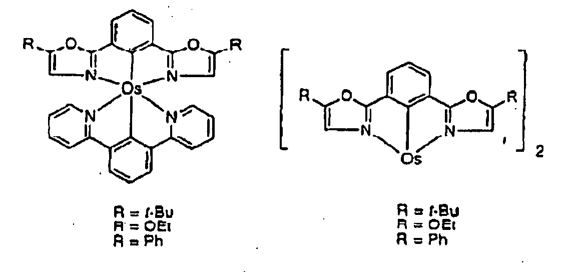

8. Ligand de formule (III) 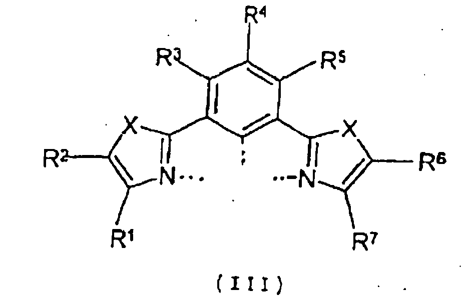 dans laquelle X représente un élément du Groupe 15 ou 16 du Tableau Périodique, R¹ à R⁷ représentent chacun, indépendamment l'un de l'autre, hydrogène, halogène, R', O-R' ou N-R'R", où R' représente un groupement hydrocarboné, pouvant éventuellement contenir des hétéroatomes, et R" représente H ou revêt une signification indiquée pour R', où deux, ou plus, groupements R¹ à R⁷ peuvent également former des noyaux condensés.

9. Composé de formule (IV) 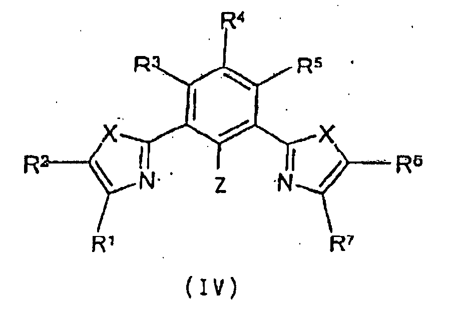 dans laquelle X représente un élément du Groupe 15 ou 16 du Tableau Périodique, R¹ à R⁷ représentent chacun, indépendamment l'un de l'autre, hydrogène, halogène, R', O-R' ou N-R'R", où R' représente un groupement hydrocarboné, pouvant éventuellement contenir des hétéroatomes, et R" représente H ou revêt une signification indiquée pour R', où deux, ou plus, groupements R¹ à R⁷ peuvent également former des noyaux condensés, et Z représente un groupement partant, en particulier H ou halogène.

10. Composé selon la revendication 9, choisi parmi

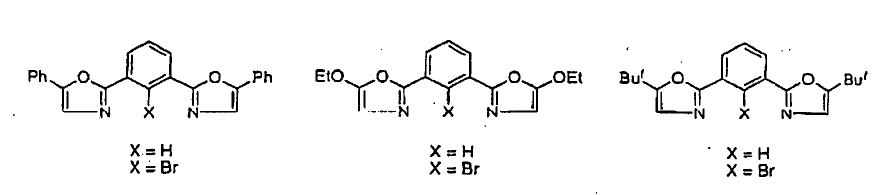

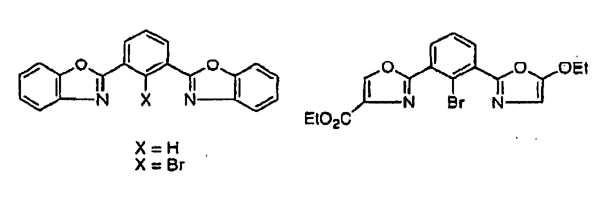

11. Dispositif photoémetteur, comprenant
(i) une anode,
(ii) une cathode et
(iii) une couche émettrice, disposée entre et en contact direct ou indirect avec l'anode et la cathode, comprenant au moins un complexe de formule (I) ou (II) 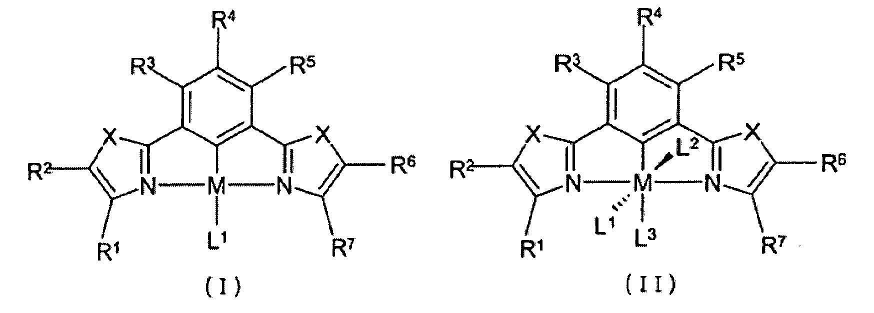 dans laquelle M est choisi parmi Mo, Tc, Ru, Rh, Pd, Ag, W, Re, Os, Ir, Pt et Au, X représente un élément du Groupe 15 ou 16 du Tableau Périodique, R¹ à R⁷ représentent chacun, indépendamment l'un de l'autre, hydrogène, halogène, R', O-R' ou N-R'R", où R' représente un groupement hydrocarboné, pouvant éventuellement contenir des hétéroatomes, et R" représente H ou revêt une signification indiquée pour R', où deux, ou plus, groupements R¹ à R⁷ peuvent également former des noyaux condensés, et L¹, L² et L³ représentent chacun, indépendamment les uns des autres, un ligand négativement chargé ou neutre, où deux, ou plus, parmi les ligands L¹, L² et L³ peuvent être reliés l'un à l'autre.

12. Dispositif photoémetteur selon la revendication 11, **caractérisé en ce qu'**il comprend en outre une couche conductrice des trous ou/et une couche conductrice des électrons.

13. Dispositif photoémetteur selon l'une des revendications précédentes 11 ou 12, **caractérisé en ce que** le complexe présent dans la couche émettrice est un émetteur de triplets.

14. Dispositif photoémetteur selon l'une des revendications précédentes 11 à 13, **caractérisé en ce que** M se trouve selon un état d'oxydation de 0 à +4.

15. Dispositif photoémetteur selon l'une des revendications précédentes 11 à 14, **caractérisé en ce que** les complexes de formule (I) ou/et (II) sont présents dans la couche émettrice selon une concentration allant de 1 à 100% en poids, sur la base du poids total de la couche émettrice.

16. Dispositif photoémetteur selon l'une des revendications précédentes 11 à 15, **caractérisé en ce que** la proportion des complexes de formule (I) dans la couche émettrice est supérieure à 80% en poids, en particulier supérieure à 90% en poids, sur la base du poids total de la couche émettrice.

17. Dispositif photoémetteur selon la revendication 16, **caractérisé en ce que** les complexes de formule (I) sont présents dans la couche émettrice sous forme d'oligomères.

18. Dispositif photoémetteur selon l'une des revendications 11 à 15, **caractérisé en ce que** la proportion des complexes de formule (I) dans la couche émettrice est supérieure à 10% en poids, en particulier supérieure à 20% en poids et jusqu'à 80% en poids, en particulier jusqu'à 70% en poids, sur la base du poids total de la couche émettrice.

19. Dispositif photoémetteur selon l'une des revendications précédentes, **caractérisé en ce qu'**il comprend, comme émetteur, le composé de
bromure de 5,5'-di-tertio-butyl-2,2'-m-phénylènebisoxazole-2-platine(II),
bromure de 5,5'-diphényl-2,2'-m-phénylènebisoxazole-2-platine(II),
bromure de 2,6-bis(5-éthoxyoxazol-2-yl)phénylplatine(II),
bromure de 2-(4-méthoxycarbonyloxazol-2-yl)-6-(5-éthoxyoxazol-2-yl)phényl-platine(II),
(3-oxo-3-éthoxypropynyl)-[2,6-bis(5-éthoxyoxazol-2-yl)phényl]platine(II),
(3-hydroxypropynyl)-[2,6-bis(5-éthoxyoxazol-2-yl)phényl]platine(II),
bromure de 5,5'-di-tertio-butyl-2,2'-m-phénylènebisoxazole-2-palladium(II),
bromure de 5,5'-diphényl-2,2'-m-phénylènebisoxazole-2-palladium(II), ou

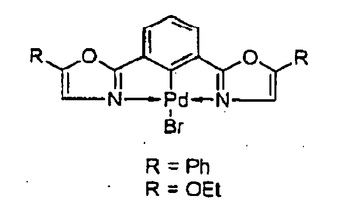

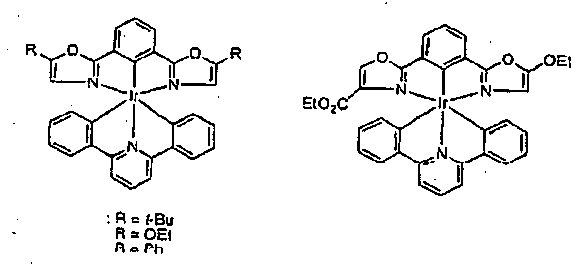

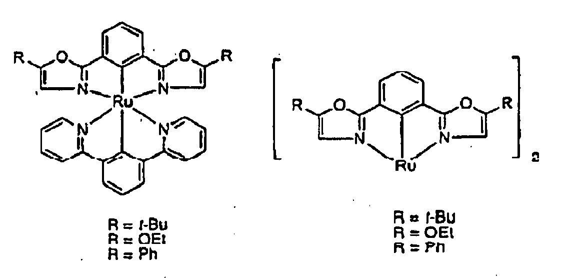

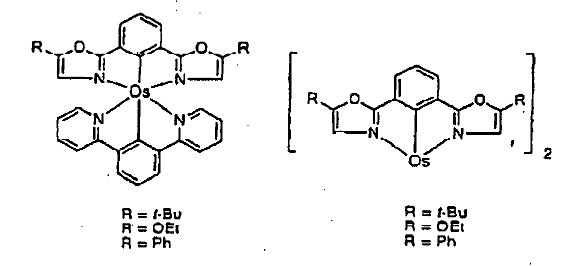

20. Dispositif photoémetteur selon l'une des revendications précédentes 11 à 19, **caractérisé en ce qu'**il comprend au moins deux complexes différents de formule (I) ou (II).

21. Dispositif photoémetteur selon l'une des revendications 11 à 15, **caractérisé en ce que** les complexes de formule (I) dans laquelle M = Pd(II) sont présents dans la couche émettrice selon une proportion supérieure à 80% en poids et les complexes de formule (I) dans laquelle M = Pt(II) selon une proportion inférieure à 10% en poids, dans chaque cas sur la base du poids total de la couche émettrice.

22. Dispositif photoémetteur selon l'une des revendications précédentes 11 à 21, **caractérisé en ce qu'**il comprend des couches cristallines ou/et quasi-cristallines comprenant des complexes de formule (I).

23. Dispositif photoémetteur selon l'une des revendications précédentes 11 à 22, **caractérisé en ce qu'**il s'agit d'une OLED.

24. Dispositif photoémetteur selon l'une des revendications précédentes 11 à 23, **caractérisé en ce qu'**il s'agit d'un afficheur ou/et d'un dispositif d'éclairage.

25. Utilisation d'un complexe de formule (I) ou (II) comme émetteur dans un dispositif photoémetteur.

26. Procédé de production d'un dispositif photoémetteur selon l'une des revendications 11 à 24, **caractérisé en ce qu'**au moins un complexe de formule (I) ou (II) est introduit dans la couche émettrice au moyen d'une sublimation sous vide.

27. Procédé de production d'un dispositif photoémetteur selon l'une des revendications 11 à 24, **caractérisé en ce qu'**au moins le complexe de formule (I) ou (II) est introduit dans la couche émettrice par des méthodes chimiques par voie humide.
